(19) Europäisches Patentamt / European Patent Office / Office européen des brevets

(11) **EP 2 890 781 B1**

(12) **EUROPEAN PATENT SPECIFICATION**

(45) Date of publication and mention
of the grant of the patent:
**23.09.2020 Bulletin 2020/39**

(21) Application number: **13756438.1**

(22) Date of filing: **29.08.2013**

(51) Int Cl.:
**C12N 5/073** (2010.01)    **G06K 9/00** (2006.01)
**C12N 5/02** (2006.01)

(86) International application number:
**PCT/EP2013/067888**

(87) International publication number:
**WO 2014/033210 (06.03.2014 Gazette 2014/10)**

(54) **AUTOMATIC SURVEILLANCE OF IN VITRO INCUBATING EMBRYOS**

EMBRYO-QUALITÄTSBEURTEILUNG BASIEREND AUF DER BLASTOZYSTENENTWICKLUNG

ÉVALUATION DE LA QUALITÉ D'UN EMBRYON SUR LA BASE DU DÉVELOPPEMENT DU BLASTOCYSTE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **30.08.2012 US 201261694915 P**
**25.06.2013 PCT/EP2013/063240**

(43) Date of publication of application:
**08.07.2015 Bulletin 2015/28**

(73) Proprietor: **Unisense FertiliTech A/S**
**8200 Aarhus N (DK)**

(72) Inventors:
• **RAMSING, Niels B.**
  **DK-8240 Risskov (DK)**
• **LÆGDSMAND, Mette**
  **DK-8830 Tjele (DK)**
• **GUNDERSEN, Jens K.**
  **DK-8260 Viby J. (DK)**
• **PORSGAARD, Søren**
  **8200 Aarhus N (DK)**
• **AGERHOLM, Inge Errebo**
  **DK-8740 Brædstrup (DK)**

(74) Representative: **D Young & Co LLP**
**120 Holborn**
**London EC1N 2DY (GB)**

(56) References cited:
**EP-A1- 2 282 210**

• HERRERO J ET AL: "Linking successful implantation with the exact timing of cell division events obtained by time-lapse system in the embryoscope", FERTILITY AND STERILITY, ELSEVIER SCIENCE INC, NEW YORK, NY, USA, vol. 94, no. 4, 1 September 2010 (2010-09-01), page S149, XP027250457, ISSN: 0015-0282 [retrieved on 2010-08-30]
• LEMMEN J G ET AL: "Kinetic markers of human embryo quality using time-lapse recordings of IVF/ICSI-fertilized oocytes", REPRODUCTIVE BIOMEDICINE ONLINE, REPRODUCTIVE HEALTHCARE LTD, GB, vol. 17, no. 3, 1 January 2008 (2008-01-01), pages 385-391, XP003028429, ISSN: 1472-6483 [retrieved on 2008-07-30] cited in the application
• M. MESEGUER ET AL: "The use of morphokinetics as a predictor of embryo implantation", HUMAN REPRODUCTION, vol. 26, no. 10, 9 August 2011 (2011-08-09), pages 2658-2671, XP055033123, ISSN: 0268-1161, DOI: 10.1093/humrep/der256 cited in the application
• MARIABEATRICE DAL CANTO ET AL: "Cleavage kinetics analysis of human embryos predicts development to blastocyst and implantation", REPRODUCTIVE BIOMEDICINE ONLINE, vol. 25, no. 5, 2 August 2012 (2012-08-02), pages 474-480, XP055079707, ISSN: 1472-6483, DOI: 10.1016/j.rbmo.2012.07.016

- **CONNIE C WONG ET AL: "Non-invasive imaging of human embryos before embryonic genome activation predicts development to the blastocyst stage", NATURE BIOTECHNOLOGY, vol. 28, no. 10, 1 October 2010 (2010-10-01), pages 1115-1121, XP055079709, ISSN: 1087-0156, DOI: 10.1038/nbt.1686**

## Description

The present invention relates to a system and a method for automatically surveying the developmental conditions of in vitro incubating embryos. The present invention may be applied within quality control of embryo handling in relation to IVF to ensure that the quality of transferred embryos is maintained. Thus, the present invention may be a tool for fertility clinics to survey and maintain a high implantation potential of in vitro incubating embryos.

## Background of invention

Infertility affects more than 80 million people worldwide. It is estimated that 10% of all couples experience primary or secondary infertility. In vitro fertilization (IVF) is an elective medical treatment that may provide a couple who has been otherwise unable to conceive a chance to establish a pregnancy. It is a process in which eggs (oocytes) are taken from a woman's ovaries and then fertilized with sperm in the laboratory. The embryos created in this process are then placed into the uterus for potential implantation.

Quality Control (QC) is an important issue in IVF clinics to monitor the quality of the treatment and the different processes in the clinics that has an effect on the developmental conditions of the embryo. Quality control can be conducted by monitoring the development of running averages in outcome variables like

- Biochemical pregnancy rates (number of HCG positive per transferred embryos)
- Fetal heats beat rates (number of fetal heart beats per transferred embryos)
- Abortion rate (number of abortions per implanted embryos)
- KID ratio (the number of known implantations (defined either as fetal heart beat or HCG positive) per known outcome)

The value of this kind of monitoring is undisputable but the monitoring is slow to respond due to a limited number of transferred embryos and an even lower amount of known implantation embryos in a specific clinic. A further problem is the time-lag due to the delay when waiting for the results of HCG test, scanning outcome and abortion information. The sensitivity is limited due to the discrete binomial outcome of the process (pregnant/not pregnant, aborted/not aborted, implanted/not implanted), which means that a large population is needed in order to evaluate if a significant change has occurred. This induces a further delay in the quality control monitoring.

Herrero J et al in "Linking successful implantation with the exact timing of cell division events obtained by time-lapse system in the embryoscope", Fertility And Sterility, Elsevier Science Inc, New York, NY, USA, vol. 94, no. 4, page S149 discloses a time-lapse system used for image acquisition of embryos and subsequent analysis of the exact timing of cell division events. The purpose of the study is to link successful implantation of embryos to the exact timing of cell division events.

Lemmen J G et al in "Kinetic markers of human embryo quality using time-lapse recordings of IVF/ICSI-fertilized oocytes", Reproductive Biomedicine Online, Reproductive Healthcare Ltd, GB, vol. 17, no. 3, pages 385 - 391 discloses a time-lapse system used to study the timing and coordination of events during early development from zygote to cleavage stage embryo. The aim was to identify markers linked to good-quality embryos and implantation. Early disappearance of pronuclei and onset of first cleavage after fertilization was correlated with a higher number of blastomeres on day 2 after oocyte retrieval. In addition, synchrony in appearance of nuclei after the first cleavage was significantly associated with pregnancy success ($P < 0.05$).

EP2282210 discloses a system and method for determining embryo quality comprising monitoring the embryo for a time period, said time period having a length sufficient to comprise at least one cell division period and at least a part of an inter-division period, and determining the length of the at least one cell division period; and/or ii) determining the extent and/or spatial distribution of cellular or organelle movement during the cell division period; and/or iii) determining duration of an inter-division period; and/or iv) determining the extent and/or spatial distribution of cellular or organelle movement during the inter-division period thereby obtaining an embryo quality measure. Thus, the selection of optimal embryos to be implanted after in vitro fertilization (IVF) is facilitated based on the timing, duration, spatial distribution, and extent of observed cell divisions and associated cellular and organelle movement.

M. Meseguer et al, "The Use of Morphokinetics as a Predictor of Embryo Implantation", Human Reproduction, no. 10, ISSN 0268-1161, page 2658 - 2671 discloses an IVF incubator with a built-in camera designed to automatically acquire images at defined time points to simultaneously monitor 72 embryos without removing the embryos from the controlled environment. Analysis of cleavage times, blastomere size and multinucleation was made for 247 transferred embryos with either failed or full implantation. Several parameters were found to be correlated with subsequent implantation (e.g. time of first and subsequent cleavages as well as the time between cleavages).

Mariabeatrice Dal Canto et al, "Cleavage kinetics analysis of human embryos predicts development to blastocyst and implantation", Reproductive Biomedicine Online, (20120802), vol. 25, no. 5, ISSN 1472-6483, Page 474 - 480, discloses that cleavage kinetics for developing embryos may be used to indicate the ability of embryos to develop to blastocyst and implant. Cleavage from 2-cell to 8-cell stage is said to occur progressively earlier in embryos with the ability to develop to blastocyst, expand and implant.

Connie Wong et al, "Non-invasive imaging of human embryos before embryonic genome activation predicts development to the blastocyst stage", Nature Biotechnology, (20101001), vol. 28, no. 10, ISSN 1087-0156, Page 1115 - 1121 discloses an approach for non-invasive imaging of human embryos and concludes the success and failure of human embryo development is largely determined by events before embryonic genome activation.

## Summary of invention

If the quality control shall pick up on e.g. failure to comply to best practice (e.g. errors in the handling of the embryos), toxicity of lab utensils or consumables (e.g. toxic oil or media) or environmental changes (contamination of laboratory air) then the response needs to be accurate, fast and sensitive. Accurate so that correcting measures are only set in place if something is really wrong, fast so that modifications can be implemented as quickly as possible and sensitive so that even small changes in the performance can be detected and corrected. Therefore, a sensitive and fast responding tool for monitoring the clinic procedures is needed.

One embodiment of the invention therefore relates to a computer implemented method according to claim 1.

It is known that the morphokinetic variables are important indicators for embryo implantation success rates. In the present invention morphokinetic parameters are utilized as quality control indicators and thereby form an alternative to monitoring the pregnancy rates or related variables which are delayed by definition. The big advantage of using morphokinetic parameters is that the number of embryos in a clinic is around one magnitude higher than the number of embryos transferred and the variables are available as soon as the incubation is ended (2-5 days after fertilization). Furthermore, the number of available morphokinetic parameters is around one magnitude higher than the number of embryos, because each embryo "produces" a plurality of morphokinetic parameters during the different developmental stages. So by monitoring the morphokinetic parameters instead of the pregnancy rates both speed and accuracy may be increased. Since e.g. the timings of cleavages are continuous variables instead of discrete the sensitivity of the statistical tests that can be used to test if two groups are significantly different are greatly increased.

The present invention is most naturally applied to human embryos, but may also be applied within monitoring of any mammal embryos.

## Definitions and embryo quality parameters

Cleavage time is defined as the first observed timepoint when the newly formed blastomeres are completely separated by confluent cell membranes, the cleavage time is therefore the time of completion of a blastomere cleavage. In the present context the times are expressed as hours post ICSI microinjection or post time for mixing of semen and oocyte in IVF, i.e. the time of insemination. This is the time of the deliberate introduction of sperm into the ovum. However, herein the term fertilization is also used to describe this timepoint. Thereby the cleavage times are as follows:

- t2: Time of cleavage to 2 blastomere embryo
- t3: Time of cleavage to 3 blastomere embryo
- t4: Time of cleavage to 4 blastomere embryo
- t5: Time of cleavage to 5 blastomere embryo
- t6: Time of cleavage to 6 blastomere embryo
- t7: Time of cleavage to 7 blastomere embryo
- t8: Time of cleavage to 8 blastomere embryo

Duration of cell cycles is defined as follows:

- cc1 = t2: First cell cycle.
- cc2 = t3-t2: Second cell cycle, duration of period as 2 blastomere embryo.
- cc2b = t4-t2: Second cell cycle for both blastomeres, duration of period as 2 and 3 blastomere embryo.
- cc3 = t5-t3: Third cell cycle, duration of period as 3 and 4 blastomere embryo.
- cc2_3 = t5-t2: Second and third cell cycle, duration of period as 2, 3 and 4 blastomere embryo.
- cc4 = t9-t5: Fourth cell cycle, duration of period as 5, 6, 7 and 8 blastomere embryo.

## Short and long cell cycles

In an embryo with a direct cleavage, the time between one of the cell divisions appear to be inadequate for DNA replication of the whole genome. For the first three cell cycles:

- Cell cycle 1, cc1, which is not a standard cell cycle as it the combines the process of fertilization, pronuclei formation and disappearance, etc. - until the advent of the first cell division at which time-point the maternal and paternal alleles have been combined in the embryonic genome. The combined genome has presumably been replicated in advance of the cell division from zygote to 2-cell. cc1=t2.

- Cell cycle 2, cc2, the time from completion of the first cell division resulting in two cells to the next cell division resulting in three cells. i.e. cc2 = t3 - t2.

- Cell cycle 3, cc3, is the time required for the third round of DNA replication. When a four cell embryo divides to five cells it should have five full copies of the genome and the two newest cells in the five-cell embryo should have successfully completed their third round of DNA replication. As the fastest cell to divide is most likely the one that initially appeared after the first cleavage of the two cell embryo to three cells, the best estimate for the fastest cell cycle is: cc3 = t5 - t3, the time of cleavage to five cells minus the time of cleavage to three cells.

*Short cell cycle embryos, SCC*

In short cell cycle embryos any of the following conditions are found:

- cc1 = t2 < 15 hr, or
- cc2 = t3 - t2 < 5 hr, or
- cc3 = t5 - t3 < 5 hr

*Long cell cycle embryos, LCC*

A similar concept, which might be equally useful is "Long cell cycle embryos". These embryos are embryos that develop very slowly with unusual long durations for their cell cycles. The long cell cycle embryos have a similar poor prognosis and are significantly less likely to implant than more normal embryos (as is shown below). However, the class of "long cell cycle embryos" is less clearly defined than the short cell cycles as the distribution at the high end is continuous with a gradually decreasing implantation potential. It is therefore less clear how to define the boundaries for this class. The boundaries for the long cell cycle embryo class has been chosen such that the LCC class correlate with a markedly reduced implantation potential similar to the one found for short cell cycle embryos. A long cell cycle class is thus defined with a comparable size to the short cell cycle class of embryos:
Long cell cycle embryo, LCC, imply

- cc1 = t2 > 32 hr, or
- cc2 = t3 - t2 > 20 hr, or
- cc3 = t5 - t3 > 25 hr

*Medium cell cycle embryos, MCC*

All embryos that fall between the groups, i.e. embryos where:

- cc1 is from 15 to 32 hrs, and
- cc2 from 5 to 20 hrs and
- cc3 from 5 to 25 hrs.

are classified as medium cell cycle embryo. These are the "normal" embryos with a normal cleavage pattern. The MCC embryos typically constitute about 60% of all embryos, which can be used to compare embryo development from clinic to clinic. E.g. the first group of embryos may be selected among MCC embryos.

Synchronicities are defined as follows:

- s2 = t4-t3: Synchrony in division from 2 blastomere embryo to 4 blastomere embryo.
- s3 = t8-t5: Synchrony in division from 4 blastomere embryo to 8 blastomere embryo.
- s3a = t6-t5; s3b = t7-t6; s3c = t8-t7: Duration of the individual cell divisions involved in the development from 4 blastomere embryo to 8 blastomere embryo.

Cleavage period: The period of time from the first observation of indentations in the cell membrane (indicating onset of cytoplasmic cleavage) to the cytoplasmic cell cleavage is complete so that the blastomeres are completely separated by confluent cell membranes. Also termed as duration of cytokinesis.

Fertilization and cleavage are the primary morphological events of an embryo, at least until the 8 blastomere stage. Cleavage time, cell cycle, synchrony of division and cleavage period are examples of morphological embryo parameters that can be defined from these primary morphological events and each of these morphological embryo parameters are defined as the duration of a time period between two morphological events, e.g. measured in hours.

A normalized morphological embryo parameter is defined as the ratio of two morphological embryo parameters, e.g. cc2 divided by cc3 (cc2/cc3), or cc2/cc2_3 or cc3/t5 or s2/cc2.

The duration of a plurality of cell cycles (e.g. CC1, CC2, CC3 and CC4) can be combined to form a common normalized parameter:

$$CC_{norm} = \sqrt{\sum_{all\ i} \left(\frac{CCi - CCi_{median}}{CCi_{median}}\right)^2}$$

where CCi e.g. is selected from CC1 to CC4. In one embodiment of the invention a high value of $CC_{norm}$ indicates a poor embryo quality as one or more of the variables CCi is far from the median, i.e. it is not the absolute values of CCi that are used, but the mutual relation of the variables. The median may be calculated based on the whole population or parts of the population (e.g. embryos with known and positive implantation). Another equivalent variable using the logarithmic value instead ($lCC_{norm}$) may also be useful in assessing embryo quality.

$$lCC_{norm} = \sqrt{\sum_{all\ i} w_i log \left(\frac{CCi - CCi_{median}}{CCi_{median}}\right)^2}$$

Likewise the synchronicity Si of the cell divisions (e.g. S2, S3 and S4) may be combined to form a common normalized parameter:

$$S_{norm} = \sqrt{\sum_{all\ i} \left(\frac{Si}{Si_{median}}\right)^2}$$

In one embodiment of the invention a high value of $S_{norm}$ indicates a poor embryo quality as one or more of the synchronicities is long compared to the. Another equivalent variable using the logarithmic value instead ($lS_{norm}$) may also be useful in assessing embryo quality.

$$lS_{norm} = \sqrt{\sum_{all\ i} w_i log \left(\frac{Si}{Si_{median}}\right)^2}$$

The variables $CC_{norm}$ and $S_{norm}$ may be calculated based on the first, second, third or fourth cell cycle, depending on the duration of the incubation.

In some examples a morphokinetic parameter for embryos which is used in accordance with certain embodiments of the invention may be established by combining a plurality of morphokinetic characteristics relating to the development of the respective embryos. For example, in some implementations the morphokinetic parameter may be derived by obtaining values for a plurality of morphokinetic characteristics relating to the development of an embryo during an observation period, for example characteristics such as cell cleavage times (t2, t3, t4...) and / or differences between subsequent cell cleavage times / divisions (t3-t2, t4-t3, t5-t4...) and / or cycle durations (cc1, cc2, cc3... etc). A value for a continuous variable may then be determined by combining differences between the obtained values for the plurality of characteristics and corresponding reference values in a pre-defined manner. The reference values may, for example, be determined from values for the plurality of characteristics obtained for one or more reference embryos of known development potential (e.g. KID positive embryos). A morphokinetic parameter for an embryo which is to be used in accordance with certain embodiments of the invention may thus be established from the continuous variable. In this respect the morphokinetic parameter may correspond with the development potential for an embryo determined in accordance with the principles set out in co-pending patent application PCT/EP2013/063240 filed 25 June 2013, the entire contents of which are incorporated herein by reference.

In accordance with some example implementations the step of combining differences between the obtained values for the plurality of morphokinetic characteristics and the corresponding reference values may take account of weighting values associated with each of the reference values. The weighting values may, for example, be statistically determined from values for the plurality of characteristics obtained for a plurality of reference embryos of known development potential. For example, the weighting values may be determined from a variance of the relevant values obtained for the plurality of reference embryos.

Some example implementations of the present invention may further comprise obtaining values for a further plurality of characteristics relating to the development of the embryo during the observation period; determining a value for a further continuous variable by combining differences between the obtained values and corresponding reference values for the further plurality of characteristics in a further pre-defined manner; and establishing the development potential for the embryo based also on the determined value for the further continuous variable.

In some examples a morphokinetic characteristic for embryos may be determined by combining / aggregating differences for various characteristics seen for each embryo and corresponding reference values (e.g. determined for a KID positive population of embryos) to generate what might be referred to as a generalized irregularity variable (GIV), which in some cases may be defined as:

$$GIV = \frac{1}{n} \sqrt{\sum_{\substack{all\ i\ in \\ sequence}} \frac{(cci - cci_m)^2}{cci_v}}$$

Where cci is a series of cell cycle durations observed for an embryo, $cci_m$ is the corresponding series of average cell cycle durations seen in a reference group of embryos (e.g. a positive KID population from patients under the age 35), and $cci_v$ are the corresponding variance values associated with the reference population. The parameter n is the number of cell cycle durations comprising the series cci. The differences ($cci - cci_m$) are normalized by the variance values $cci_v$ as part of the combining. This means that differences for particular cell cycles (values of i) which exhibit relatively high variance in the sample population contribute less to the value of GIV than differences for cell cycle which exhibit relatively low variance in the sample population. The differences ($cci - cci_m$) are squared in the combining, and this means the contribution to GIV is the same for a given difference, regardless of whether it is positive or negative (i.e. whether cci is longer or shorter than $cci_m$). The series cci may comprises durations for various different cell cycles in different implementations as discussed further below.

Generally speaking GIV as defined above is low when the study embryo exhibits a regular cleavage pattern and is high when the embryo exhibits an irregular cleavage pattern.

In some examples a morphokinetic characteristic for embryos may be referred to as a generalized time variable (GTV) defined as:

$$GTV = \frac{1}{k} \sum_{\substack{all\ j\ in \\ sequence}} \frac{\Delta tj - \Delta tj_m}{\Delta tj_v}$$

Where $\Delta tj$ is a series of differences in times for subsequent cell divisions observed for an embryo, $\Delta tj_m$ is the corresponding series of average values seen in a reference group of embryos (e.g. the positive KID population from patients under the age 35), and $\Delta tj_v$ are the corresponding variance values associated with the reference population. The parameter k is the number of values comprising the series $\Delta tj$. The differences ($\Delta tj - \Delta tj_m$) are normalized by the variance values $\Delta tj_v$ as part of the combining. This means that differences for particular cell divisions (values of j) which exhibit relatively high variance in the sample population contribute less to the value of GTV than for those which exhibit relatively low variance in the sample population. In this example the contribution to GTV for each time difference depends on whether the difference in times for a particular pair of subsequent cell divisions is faster or slower than the average seen in the positive KID population (i.e. whether the difference is positive or negative).

The pending application PCT/DK2012/050236 filed at 29.06.2012 and entitled "Adaptive embryo selection criteria optimized through iterative customization and collaboration" from the same applicant relates to the issue of adapting embryo quality criteria across populations of embryos cultures under different incubation conditions, e.g. in different clinics. This application is hereby incorporated by reference in its entirety. However, quality parameters like $CC_{norm}$, $ICC_{norm}$, $S_{norm}$ and $IS_{norm}$ may help to ensure that quality models will be directly applicable across different populations of embryos cultured under different incubation conditions, because they are based on variables that are insensitive to differences in running conditions. Another example of that is quality parameters based on relative time periods (e.g. CC2/CC3), variables divided with a central estimate of that variable (e.g. mean or median, e.g. cc2/cc2_median) or using target intervals where the center is scaled according to a central estimate and the boundaries are scaled according to a variance estimate (e.g. variance, standard deviation, percentiles).

The following discrete (binary) variables can be used

- MN2: Multi nucleation observed at the 2 blastomere stage; can take the values "True" or False".
- MN2val: the number of multinuclear cells at the 2 cell stage (0,1,2).
- MN4: Multi nucleation observed at the 4 blastomere stage; can take the values "True" or False".
- MN4val: the number of multinuclear cells at the 4 cell stage (0,1,2,3,4).
- EV2: Evenness of the blastomeres in the 2 blastomere embryo; can take the values "True" (i.e. even) or "False" (i.e. uneven).

*Blastocyst related parameters*

A blastocyst quality criterion is an example of an embryo quality criterion. The following blastocyst related parameters may be used:

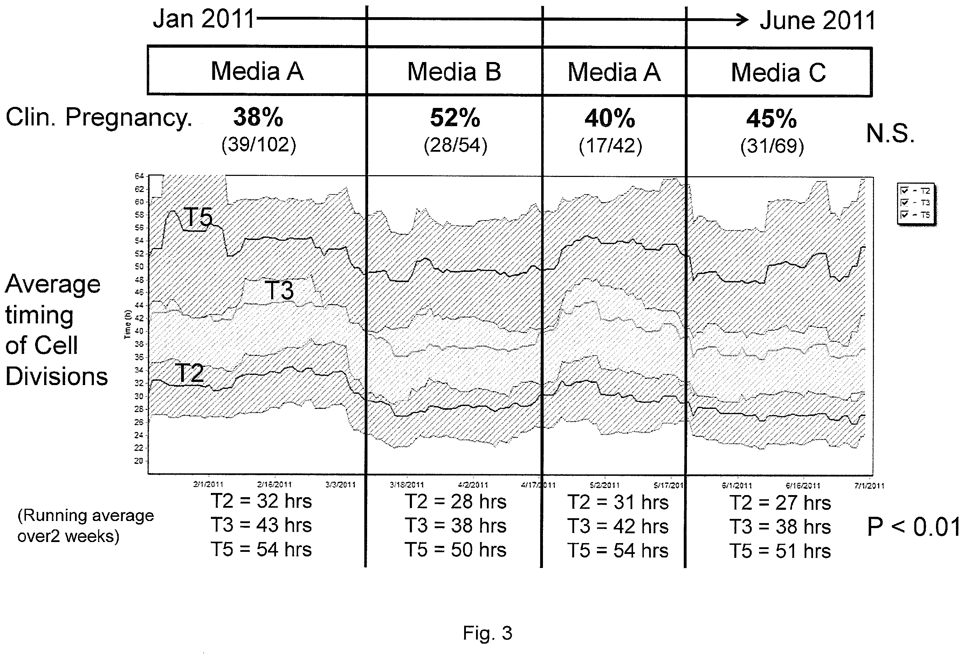

Initial compaction (IC) describes the first time a compaction between 2 or more blastomeres is observed. Compaction is a process wherein an intensification of the contacts between the blastomeres with tight junction and desmosomes result in reduction of the intercellular space and a blurring of the cell contours (see fig. 3). Compaction can be seen at the 6-8 cell stage but rarely do the embryos cleave to 16 cell or more before compaction occurs.

Compaction/Morula (M) is defined as the first time where no plasma-membrane between any blastomeres are visible. When compaction is complete no plasma-membranes between any of the blastomeres forming the compaction are visible and the embryo can be defined as a morula. The stage is characterized by a process with an intensification of the contacts between the blastomeres with tight junction and desmosomes resulting in reduction of the intercellular space and a blurring of the cell contours. Compaction/Morula can sometimes be seen at the 6-8 cell stage during the 3'rd division (synchrony) period (S3) but most often compaction/Morula is seen after S3 close to or right in the beginning of the fourth synchrony period (S4). Rarely do the embryos cleave to 16 cell or more before compaction occurs.

Initial differentiation of trophectoderm (IDT) is defined as the first time during the compaction stage where distinct trophectoderm cells are recognized. It describes the onset of differentiation of the trophectoderm cells. The blastomeres gradually become flattened and elongate creating a barrier between the outside environment and the inner cell part of the morula.

Onset of cavitation/early blastocyst/blastocyst (BI) is defined as the first time a fluid-filled cavity, the blastocoel, can be observed. It describes the initiation of the transition period between the compaction and the blastocyst stage of the embryo. Embryos often remain in this transition stage for a period of time before entering the actual blastocyst stage. Onset of cavitation usually appears immediately after differentiation of the trophectoderm cells. The outer layer of the morula with contact to the outside environment begins to actively pump salt and water into the intercellular space, as a result of which a cavity (the blastocoel) begins to form.

Initial differentiation of inner cell mass (IDCIM) defined as the first time after OC (onset of cavitation) the inner cell mass can be recognized. IDCIM describes the initiation of inner cell mass development. An eccentrically placed cluster of cell connected of gab junction where the boundaries between the cells seem not well defined.

Onset of expansion of the blastocyst (EB) is defined as the first time the embryo has filled out the periviteline space and starts moving/expanding Zona Pelucidae. EB describes the initiation of the embryos expansion. As the blastocyst expands the zona pellucida becomes visibly thinner.

Hatching blastocyst (HB) is defined as the first time a trophectoderm cell has escaped / penetrated the zona pellucida.

Fully hatched blastocyst (FH) is defined as when hatching is completed with shedding zona pellucida.

Number of Contractions (NC (X)) describes the number of contractions (X) the embryo undergoes after the onset of cavitation. In many embryos the contractions can be quite large and lead to a large reduction of the embryonic volume. A contraction is defined as a reduction in the cross sectional surface area of the embryo of more than 15%.

Degree of vacuolization (VC (X): X={0, 1, 2, 3}) describes the extent of vacuolization after the embryo has undergone compaction. The degree of the vacuoles is rated by a 0-3 scale (0 = no vacuolization; 1 = small degree of vacuolization where small vacuoles appear after compaction but the embryonic development does not appear to be affected; 2 = moderate degree of vacuolization where large vacuoles appear after compaction and embryonic development is affected to some extent; 3 = severe vacuolization where very large vacuoles appear after compaction and embryonic development is severely affected. In this incidence the vacuoles can be mistaken for the blastocyst cavitation.

Compaction - de-compaction-compaction (CDC) describes a phenomenon where an onset compaction of the embryo had started but becomes disrupted of cleavage. The cell boundaries of the blastomeres become visible again but after a while the embryo returns into a compacted composition.

<u>Partial Compaction (PC)</u> describes an uneven compaction where one or more of the blastomeres are not included in the compaction.

In humans embryonic gene activation (EGA) typically occurs on day 3, around the 8-cell stage. Before EGA embryos are observed to translate only maternally inherited mRNA, i.e. that mRNA which is present in the oocyte when it is fertilized. The mRNA is localized in different parts of the oocyte, so that as the oozyte/zygote divides it is segregated into different blastomeres. This segregation is thought to underlie much of the differentiation of cells that occurs before EGA. After EGA the embryo begins to transcribe its own DNA, cells become motile and cell division becomes asynchronous. Since the cells are now transcribing their own DNA, this stage is where differential expression of paternal genes is first observed. The transition around EGA is also referred to as midblastula or midblastula transition.

## Rearrangement of cellular position = Cellular movement (see below)

Cellular movement: Movement of the center of the cell and the outer cell membrane. Internal movement of organelles within the cell is NOT cellular movement. The outer cell membrane is a dynamic structure, so the cell boundary will continually change position slightly. However, these slight fluctuations are not considered cellular movement. Cellular movement is when the center of gravity for the cell and its position with respect to other cells change as well as when cells divide. Cellular movement can be quantified by calculating the difference between two consecutive digital images of the moving cell. An example of such quantification is described in detail in the pending PCT application entitled "Determination of a change in a cell population", filed Oct 16 2006. However, other methods to determine movement of the cellular center of gravity, and/or position of the cytoplasm membrane may be envisioned e.g. by using FertiMorph software (ImageHouse Medical, Copenhagen, Denmark) to semiautomatically outline the boundary of each blastomere in consecutive optical transects through an embryo.

*Other parameters*

Organelle movement: Movement of internal organelles and organelle membranes within the embryo which may be visible by microscopy. Organelle movement is not Cellular movement in the context of this application.

Movement: spatial rearrangement of objects. Movements are characterized and/or quantified and/or described by many different parameters including but restricted to: extent of movement, area and/or volume involved in movement, rotation, translation vectors, orientation of movement, speed of movement, resizing, inflation/deflation etc. Different measurements of cellular or organelle movement may thus be used for different purposes some of these reflect the extent or magnitude of movement, some the spatial distribution of moving objects, some the trajectories or volumes being afflicted by the movement.

Embryo quality is a measure of the ability of said embryo to successfully implant and develop in the uterus after transfer. Embryos of high quality have a higher probability of successfully implant and develop in the uterus after transfer than low quality embryos. However, even a high quality embryo is not a guarantee for implantation as the actual transfer and the woman's receptivity highly influences the final result.

Viability and quality are used interchangeably in this document. Embryo quality (or viability) measurement is a parameter intended to reflect the quality (or viability) of an embryo such that embryos with high values of the quality parameter have a high probability of being of high quality (or viability), and low probability of being low quality (or viability). Whereas embryos with an associated low value for the quality (or viability) parameter only have a low probability of having a high quality (or viability) and a high probability of being low quality (or viability)

**Description of Drawings**

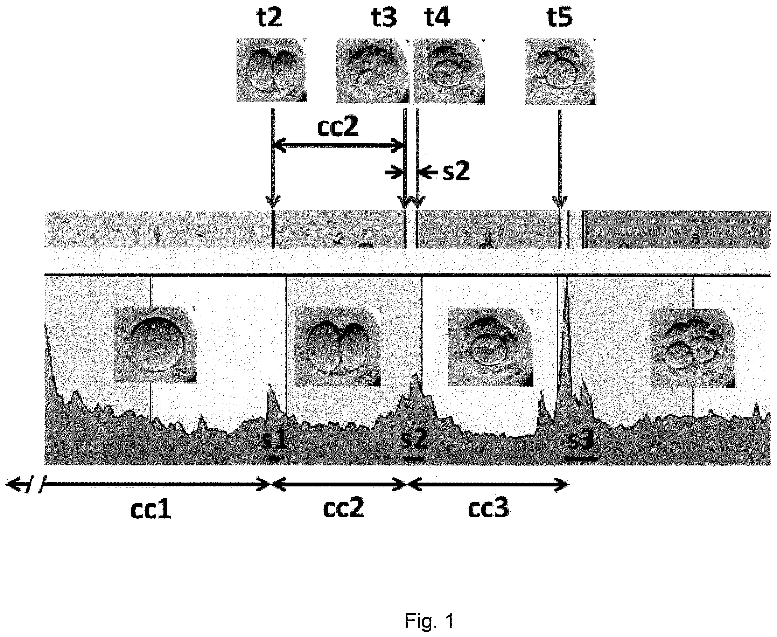

Fig. 1. Nomenclature for the cleavage pattern showing cleavage times (t2-t5), duration of cell cycles (cc1-cc3), and synchronies (s1-s3) in relation to images obtained.

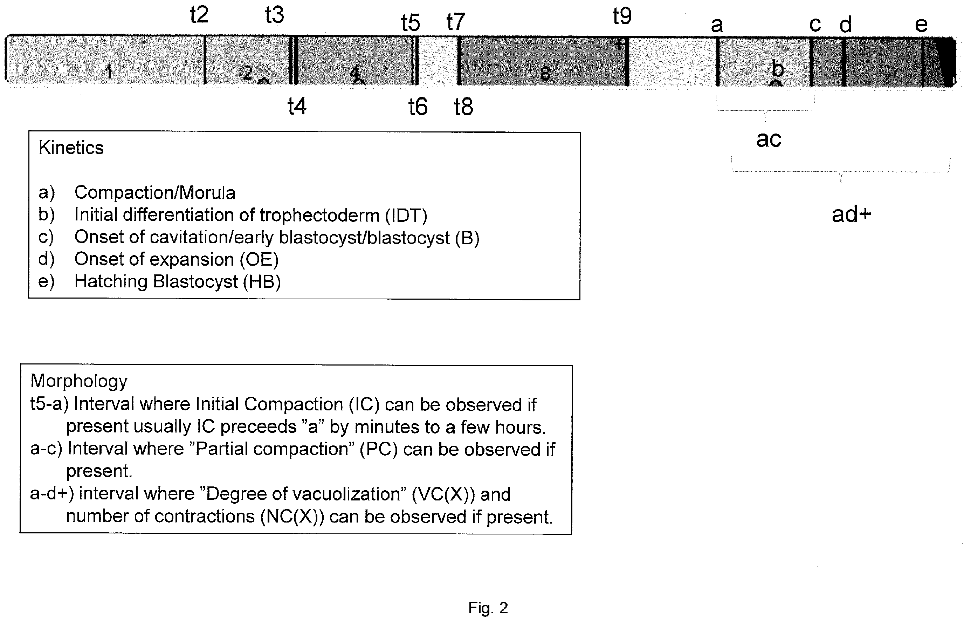

Fig. 2: The development of the embryo until the blastocyst stage. The number refers to the number of blastomeres in each stage. The letters a to e refer to the following kinetic parameters. a: Compaction/Morula (M), b: Initial differentiation of trophectoderm (IDT), c: Onset of cavitation/early blastocyst/blastocyst (BI), d: Onset of expansion of the blastocyst (EB), e: Hatching Blastocyst (HB). Initial Compaction (IC) can be observed between t5 and compaction/morula (a), if present usually IC precedes compaction/morula by minutes to a few hours. Partial compaction (PC) can be observed between stages a and c if present. Vacuolization" (VC(X)) and contractions (NC(X)) can be observed between stages a and d+ if present.

Fig. 3 shows the variation of morphokinetic parameters (in this case t2, t3 and t5) as a function of the culture medium in a fertility clinic. The total period runs from February 2011 to June 2011. Of the three media used (A, B, C) media A provided the worst embryo development (latest cell division timing and t2, t3 and t5 are all higher for media A). Media A also provided worse implantation rates and pregnancy rates. Media B and Media C both provided normal embryo development and high implantation and pregnancy rates. Applying the present invention to surveillance of morphokinetic parameters of embryos developing in different media can reveal these problems online as they progress.

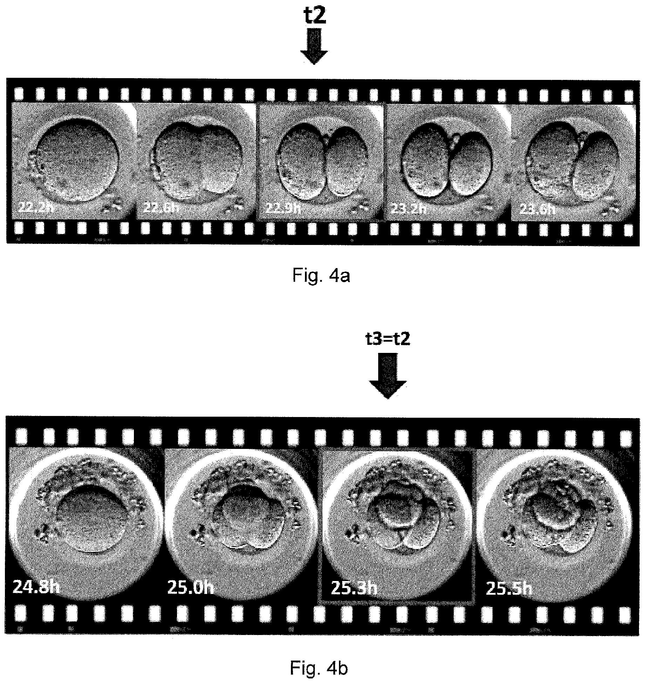

Fig. 4a. A series of images showing where the time of t2 (time of cleavage where a 2 blastomere embryo is created, i.e. the time of resolution of the cell division) is seen to happen at 22.9 hours.

Fig. 4b. A series of images showing direct cleavage to a 3 blastomere embryo. Cleavage from 1 to 3 cells happens in one frame, thus t3 = t2 and CC2 is thereby less than 5 hours, i.e. an example of an SCC embryo.

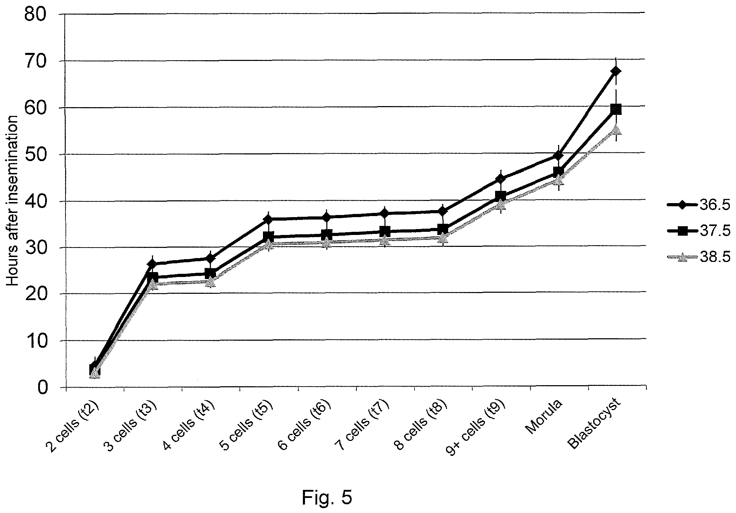

Fig. 5. Mouse embryo development with varying temperature of the incubation medium (see example 1).

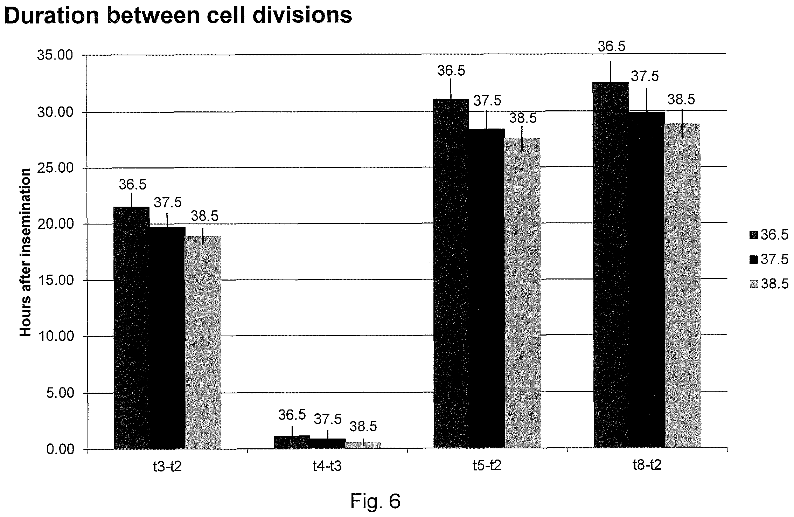

Fig. 6. Duration between various cell divisions for mouse embryos for varying temperatures of the incubation medium (see example 1).

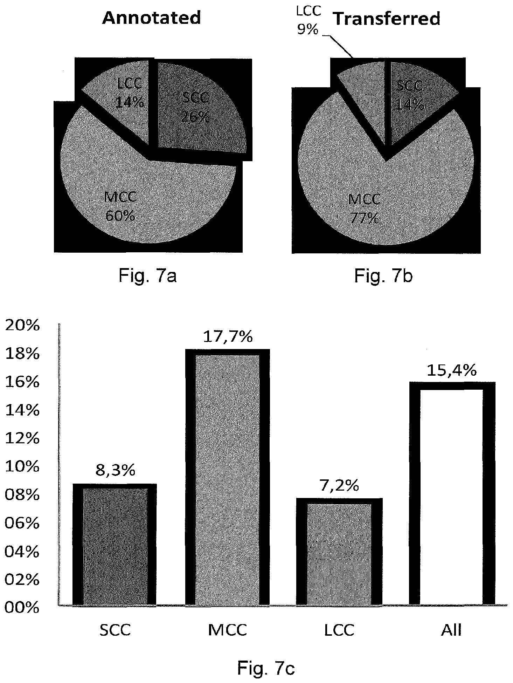

Figs. 7a-c. Direct cleavage has been analysed in a large data material containing 4,181 IVF treatments from eight IVF clinics which involve a total of 32,382 retrieved oocytes that were inseminated and placed in EmbryoScope incubators. 18,024 annotated embryos have been evaluated of which 5491 were transferred. As shown in fig. 7a short cell cycles were found in 4709 of the 18024 annotated embryos (26.1 %) and long cell cycles in 2464 of 18024 embryos (13.7%). The remaining 10851 embryos (60.2%) were MCC. The abundance of both SCC and LCC was reduced among the transferred embryos: SCC 14.1% (776 of 5491) and 9.2% (503/5491) as shown in fig. 7b. The remaining 76.7% was "normally" dividing MCC embryos. The KID ratio is shown in fig. 7c. The two aberrant classes SCC and LCC display a significantly reduced KID ratio. The relative implantation ratio was reduced to 8.3%/17.7% = 47% of the MCC rate for SCC embryos and 7.2%/17.7% = 41% of the MCC rate for LCC embryos. In one embodiment of the invention SCC and/or LCC embryos are therefore defined as embryos with morphokinetic parameter outliers. Further, variation in the relative amount of SCC and/or LCC embryos may be an indicator of change in culturing conditions and by monitoring this variation early warnings may be provided.

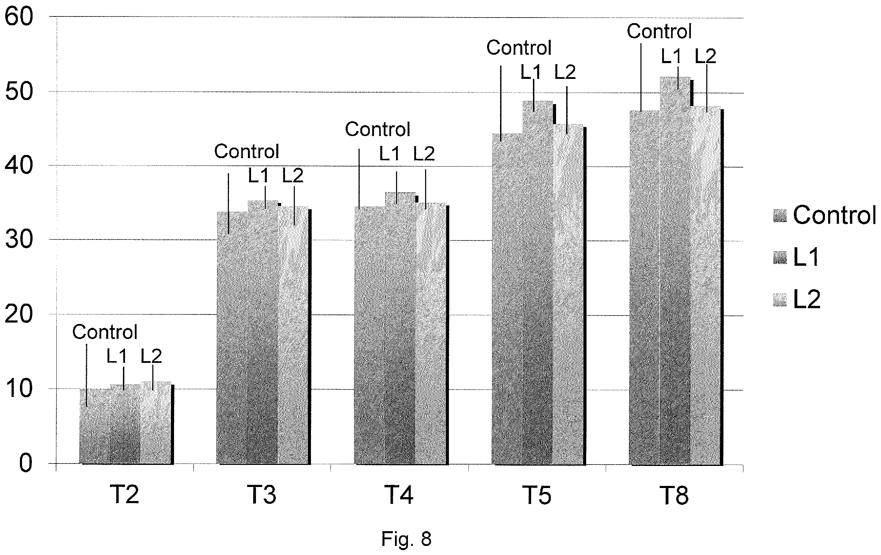

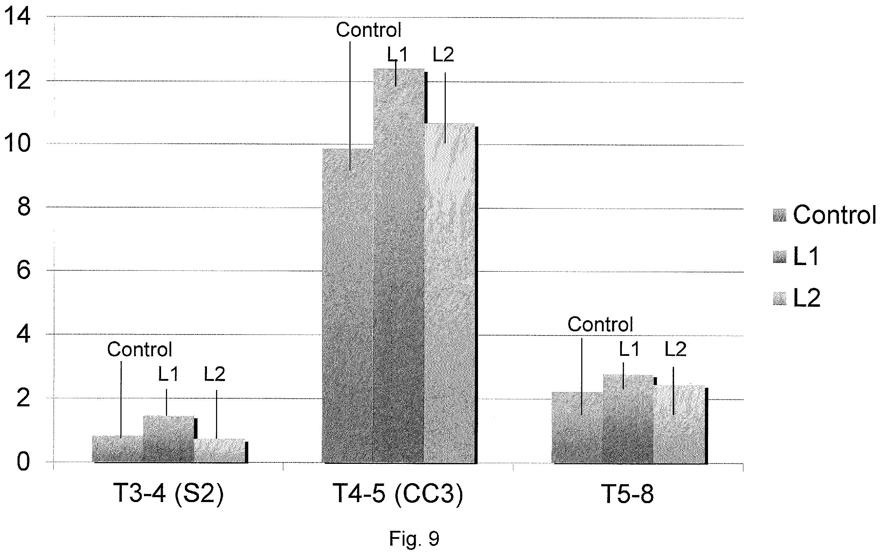

Figs. 8 and 9 show the variation in the average of the morphokinetic parameters t2, t3, t4, t5, t8, s2, cc3 and t8-t5 for embryos cultures in different lots of oil. The differences are clearly seen and especially oil lot "L1" is significantly different, possibly due to a small amount of a toxic substance that leads to markedly lower embryo quality. The embryos cultured in L1 are seen to develop more slowly and with a longer cell cycle (cc3). For the cleavage times the difference is more pronounced for the latest morphokinetic parameters t5 and t8. Thus, monitoring the morphokinetic parameters is a sensitive and fast responding tool to survey the quality of incubating embryos.

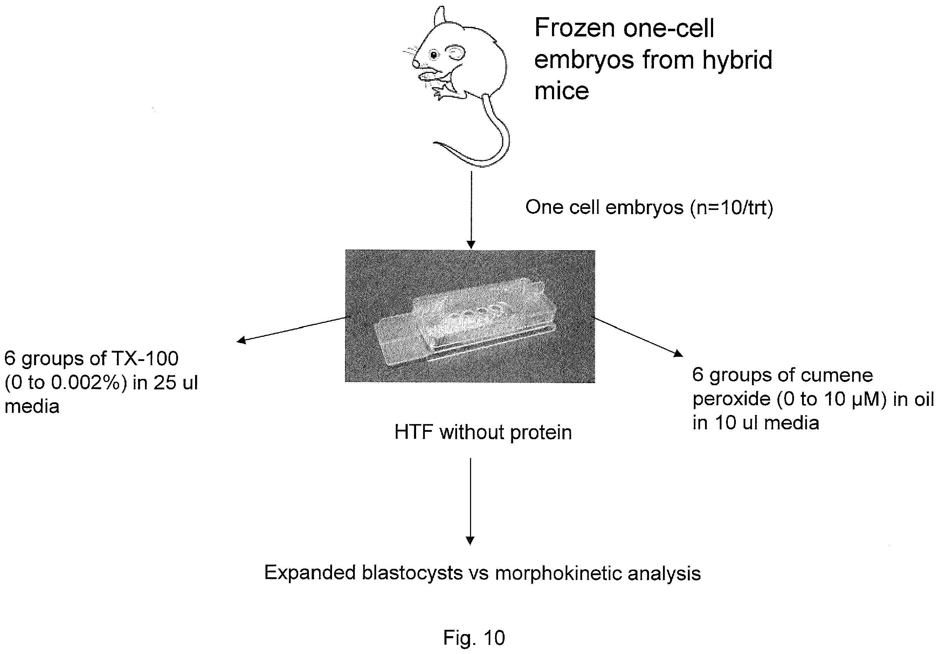

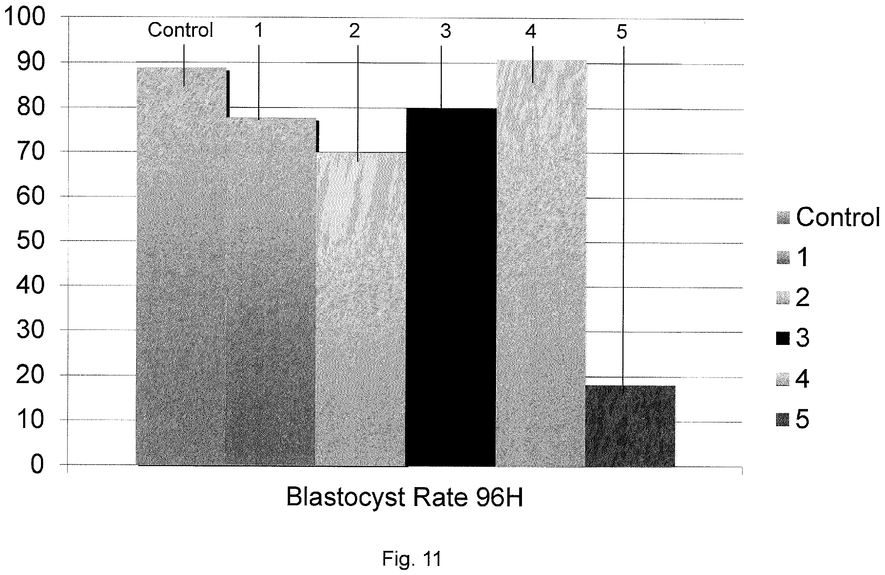

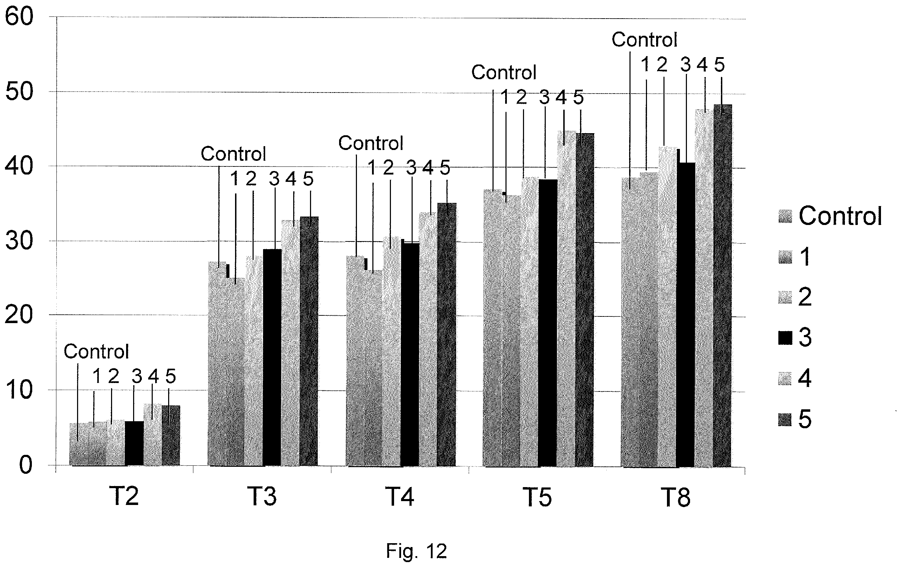

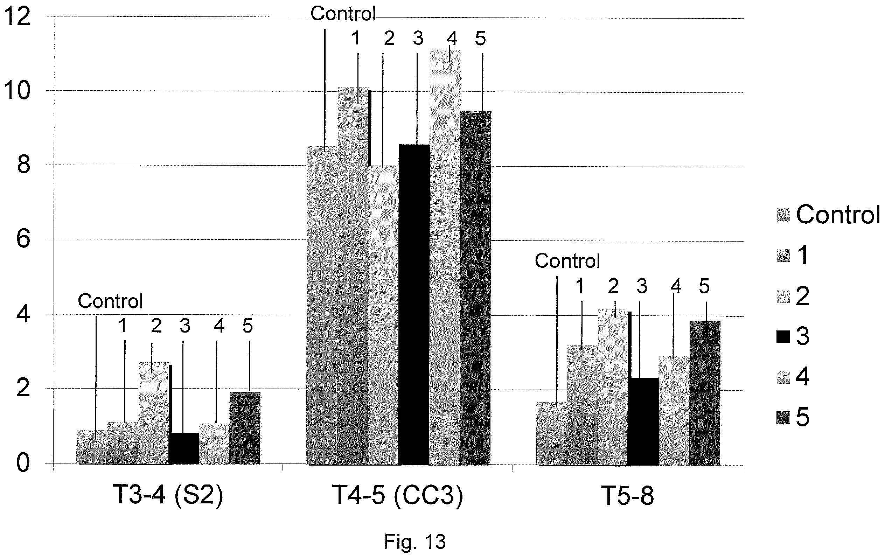

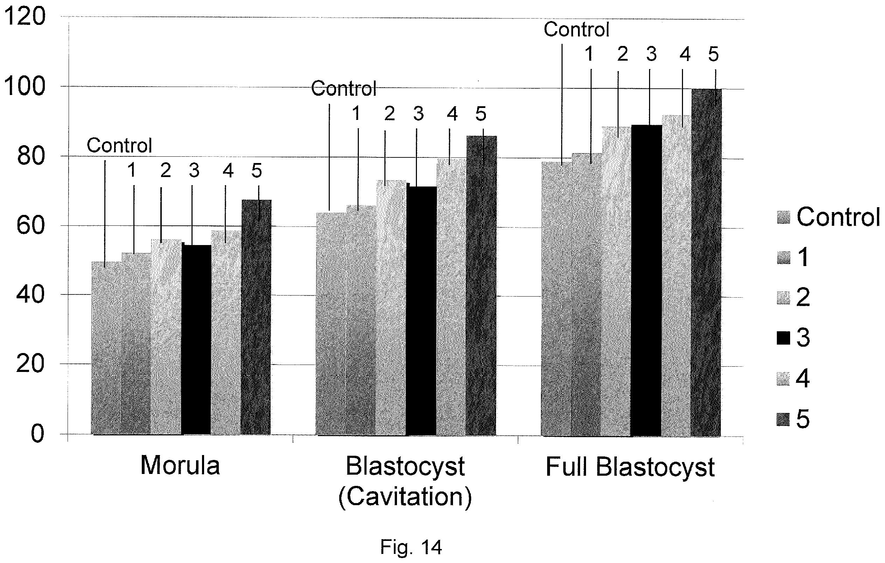

Figs. 10-14. Experiments were conducted with controlled amounts of TX-100 (Triton X-100) in culture media for embryos from hybrid mice. The leftmost columns are the control group with no TX-100 and the five other groups have increasing amounts of TX-100, up to 0.002%. Fig. 11 shows the blastocyst rate at 96 hours, i.e. the rate of embryos having reached the blastocyst stage at 96 hours. Only group 5 with most TX-100 is significantly different. Fig. 12 shows the variation of t2, t3, t4, t5 and t8 across the groups and a trend towards a slower development with increasing amounts of TX-100 is seen with the most pronounce differences at the later cleavage times. Fig. 13 shows the variation of s2, cc3 and t8-t5 across the groups with no clear trends other than most groups have higher values than the control group. Fig. 14 shows the variation for the timings of different blastocyst stages and a clear trend towards a slower development with increasing amounts of TX-100 is seen.

**Detailed description of the invention**

A way to identify a viable embryo in a cohort of embryos from an IVF treatment would be to compare the recorded temporal pattern of cell division, represented by the morphokinetic parameters, to the recorded temporal patterns of cell division from embryos in past treatment cycles. A viable embryo would be characterized by having morphokinetic parameters that match the recorded morphokinetic parameters from embryos that implanted and resulted in a live birth in the past. In selecting the embryo for transfer that display morphokinetic parameters resembling those of positive embryos (i.e. embryos from ongoing or successfully completed pregnancies) and differ where possible from the majority of negative embryos (i.e. those embryos that failed to implant or gave rise to clinical abortions) it would be possible to improve the likelihood of obtaining a pregnancy and to achieve the desired outcome of the fertility treatment. The present invention turns this known principle upside down: By monitoring the morphokinetic parameters undesirable differences or trends may be detected much sooner and this may give rise to early warnings pointing to e.g. unintended differences in embryo handling. On the other hand morphokinetic parameter surveillance may also be used to alleviate fears after multiple implantation failures, because morphokinetic parameter analysis can show that embryo development is indeed normal.

Several factors have been shown to effect embryo development, e.g. the timing of cell divisions. The factors that have been shown to influence embryo development, and consequently the derived morphokinetic parameters, include: Temperature, media composition, pH, $CO_2$ and oxygen, growth factors, cultivation vessel etc. Other factors such as patient age, etiology, BMI, stimulation protocol (agonist/antagonist, type of hormone rFSH/hMG), embryo handling (pipettes, fertilization method, assisted hatching, removal of blastomeres, polar bodies or trophectoderm cells by biopsy), have been proposed by various scientists to influence embryo development and in particular the timing of cellular events such as cell cleavage.

Thus, one embodiment of the invention applies within quality control in a fertility clinic by comparing average cleavage patterns of embryos in recent treatment cycles with cleavage patterns from past cycles. Temporal changes in general morphokinetic parameters for good quality embryos may indicate an unintended change in protocol, such as bad lot of media, problems with incubators, pipette tips, etc.

For continuous monitoring the steps of the claimed method can be repeated and the second dataset can thus be continuously updated with the most recent embryo data, such as the most recent embryo data selected from a specific time period or selected from a predefined number of the most recent embryos, such as the most recent embryos from a predefined time period, such as number of hours, days, weeks or months. Said predefined number of embryos or predefined time period may advantageously be determined as a user input. The culturing conditions can thereby be continuously surveyed.

The method is computer implemented and thus the functionality of issuing a warning when the morphokinetic difference is above a predefined level may advantageously be implemented. The type of the warning may be dependent of the severity of the detected difference, e.g. green light for normal, yellow light for possible problems and red light for a serious quality issue.

The first group of embryos will typically be a reference group or control group and the second group of embryos may be the group of embryos that are monitored and compared to the control group. The detected morphokinetic difference between the groups of embryos can be determined by standard statistical methods known in the art, e.g. the predefined level for issuing a warning may be determined as a predefined level above the standard deviation of one or more of the morphokinetic parameters from the first dataset.

Morphokinetic parameter outliers may be defined as relative outliers, i.e. the outermost 3% of the population. However, it may be an advantage to define morphokinetic parameter outliers as absolute outliers, i.e. a morphokinetic parameter is defined as an outlier if it outside a predefined absolute number. This is the case for SCC embryos and LCC embryos, as defined herein, that are examples of absolute outliers. The lower limit of 5 hours for the SCC embryos comes from the fact that 5 hours is not enough time for DNA replication of the whole genome.

The morphokinetic parameter outliers may be excluded from the first datasets and/or from the second dataset. Monitoring the variation in the morphokinetic parameters may be a good indicator for quality issues as many of the morphokinetic parameters are quality parameters for the embryo quality. However, it may be an advantage to monitor the number or frequency or distribution of morphokinetic outliers, in particular in the second dataset, as any variation in the outliers may be an indicator of quality issues.

Only the specific morphokinetic parameters that are outliers may be excluded from the dataset. However, it may be an advantage to exclude all data from specific embryos that have one or more morphokinetic outlier in their development.

It may be that the second group of embryos is a subset of the first group of embryos such that the second dataset is a subset of the first dataset.

The method according to any of the preceding claims, wherein the morphokinetic parameters are selected from the group of:

the time of cleavage to a n blastomere embryo, tn, where n = {1, ..., 8},
duration of cell cycles cc1, cc2, cc2b, cc3, cc2_3 and cc4,
synchronicities s2, s3, s3a, s3b and s3c,
number of blastomeres at specific predefined timepoints,
timing, extent or duration of cellular and/or organelle movement,
any combination of these morphokinetic parameters.

The following may be selected as a morphokinetic parameter:

$$CC_{norm} = \frac{1}{n}\sqrt{\sum_{all\ i}\left(\frac{CCi - CCi_m}{CCi_v}\right)^2}$$

wherein i = {1,2,3,4}, $cci_m$ is the mean value or median value of cci and $cci_v$ is the variance of cci.

In one embodiment of the invention the first and/or second group of embryos are embryos that has been fertilized, preserved and/or incubated under a specific set of conditions. The first group of embryos may have been fertilized under a different set of conditions than the second group of embryos. These conditions may be selected from the group of: type of fertilization treatment, preservation such as cryopreservation, incubating temperature, type of media, specific incubator, specific treatment such as hormonal treatment, hormonal treatment, aspiration, male factor, specific incubator, incubation temperature, type of media, type of oil, PGD Treatment, transfer, cryopreservation, thawing, time outside incubator, number of fertilization treatments.

In one embodiment of the invention the second group of embryos are embryos that originate from predefined embryo donors. The predefined embryo donors may be selected from the group of: individuals younger or older than a predefined age, embryo donors in a specific treatment or stimulation protocol, embryo donors in a specific fertilization treatment, embryo donors with a specific diagnosis such as hereditary chromosomal diseases, Hiv, Hep, PCO's, individuals with current or past exposure to radiation or hazardous chemical substances, individuals with current or past drug use, individuals with a BMI higher or lower than a predefined level, smokers, non-smokers, individuals with normal or abnormal menstrual cycle.

The second group of embryos may be selected as embryos from specific patient groups (e.g. younger patients), patients with specific diagnosis (e.g. endometriosis), specific fertilization treatments (e.g. ICSI), embryos that has received special pretreatment (e.g. cryopreserved).

A specific problem arises if the clinic occasionally has patients with very deviating cleavage patterns. Then the number of these patients may strongly influence the average of the timings. I this case monitoring of the above mentioned variables on selected groups of embryos may be a solution.

The lengths of the cell cycles cci (cc1, cc2, cc3 and cc4) are all important variables in determining the quality of the embryo. Combinations of the morphokinetic parameters may be used to construct a new morphokinetic parameter that is resilient to the rarely occurring extremes in timing of the specific cleavage events

$$CC_{norm} = \frac{1}{n}\sqrt{\sum_{all\ i}\left(\frac{CCi - CCi_m}{CCi_v}\right)^2}$$

Where the $CCi_m$ is the median (average) value of cci and $cci_v$ is the variance of cci. These can e.g. be calculated on the group of KID positive embryos, from all transferred embryos or from all embryos. If a maximum limit x is set for $CC_{norm}$ (e.g. 3) it means that if the CCi's from the second group of embryos on average fall more than x standard deviations (of the KID positive, transferred or all) from the average value of the first group of embryos (e.g. KID positive, transferred or all) that data will be excluded.

To monitor that best laboratory practice is being observed it will be possible to isolate embryos that have undergone a specific treatment to monitor the variables for these embryos to see if there is a change over time, i.e. the second group of embryos is selected among embryos with this specific treatment. Also groups of embryos that have been handled by a specific person of the laboratory or clinic staff can be selected as the second group of embryos to evaluate if changes has occurred over time or if there is a general difference between staff performance.

In a further embodiment of the invention the first group of embryos comprise preimplantation data from implanted embryos that have resulted in ongoing pregnancies, live born babies, fetal heart beat (FHB), and/or gestational sacs. I.e. the first group is selected to reflect high quality embryos with proven track record.

In yet another embodiment of an embryo dataset (e.g. a first or second embryo dataset) comprise morphokinetic parameters for

1) all embryos in a group of monitored embryos, or
2) a functionally defined subgroup from the group of embryos.

I.e. all embryos in group of monitored embryos (i.e. all embryos ever monitored in a certain clinic) can be selected as the frame of reference for the statistical calculations. Or just a subgroup is selected where this subgroup is functionally defined. Examples of functionally defined subgroups:

- all fertilized embryos in the group,
- embryos that have divided to at least a predefined number of cells at a predefined number of hours after insemination, such as divided to at least 7 cells 68 hours after insemination,
- embryos that have less than a predefined percentage of fragmentation at a predefined hours after insemination, e.g. less than 20% fragmentation 68 hours after insemination,
- embryos that are not multinucleated at a certain cell stage, e.g. at the four cell stage,
- embryos that have been classified as "Good quality embryos" (GQE) by a qualified embryologist,
- embryos that have been chosen for freeze or transfer,
- embryos that have been chosen for transfer, and/or
- embryos that have implanted.
- Embryos selected by excluding poorly developing embryos, e.g. by excluding Scc and/or Lcc embryos or by employing other exclusion criteria as e.g. described in pending applications PCT/DK2012/05018 or EP 12174432.0, the latter filed at 29.06.2012 and entitled "Embryo quality assessment based on blastocyst development".

In a further embodiment of the invention the morphokinetic parameters are selected from the group of:

- the timing and/or duration cell-division periods and inter-division periods,
- the timing and/or duration of: cleavage times, cleavage periods and cell cycle times;
- the timing and/or duration of divisional stages and quiet stages,
- synchrony of cell divisions;
- timing, extent or duration of cellular and/or organelle movement,
- timing, extent or duration of quality criteria, such as quality criteria as described in PCT/DK2012/05018
- Blastocyst quality criteria as described in EP 12174432.0

In a further embodiment of the invention the morphokinetic parameters are selected from the group of:

- the timing and/or duration cell-division periods and inter-division periods, determined for the first, second, third, fourth, fifth and/or sixth cell division;
- the timing and/or duration of: cleavage times, cleavage periods and cell cycle times determined for the first, second, third, fourth, fifth and/or sixth cell division;
- the timing and/or duration of divisional stages and quiet stages determined for the first, second, third, fourth, fifth and/or sixth cell division;
- synchrony of the second and third cell division;
- timing, extent or duration of cellular and/or organelle movement determined for the first, second, third, fourth, fifth and/or sixth cell division;
- timing, extent or duration of cellular and/or organelle movement determined in between the first, second, third, fourth, fifth and/or sixth cell division;

In a further embodiment of the invention said second group of embryos dataset is substantially smaller than the first group of embryos, such as 2 times smaller, such as 5 times smaller, such as 10 times smaller, such as 50 times smaller, such as 100 times smaller, such as 200 times smaller, such as 500 times smaller, such as 1000 times smaller.

In a further embodiment of the invention the embryos are cultured and/or monitored in an incubator. Preferably the embryos are monitored through image acquisition, e.g. by means of time-lapse microscopy equipment, such as image acquisition at least once per hour, preferably image acquisition at least once per half hour such as image acquisition at least once per twenty minutes, such as image acquisition at least once per fifteen minutes, such as image acquisition at least once per ten minutes, such as image acquisition at least once per five minutes, such as image acquisition at least once per two minutes, such as image acquisition at least once per minute.

The method according to the invention may be computer implemented or at least partly computer implemented thereby providing an efficient customizable tool for fertility clinics. I.e. the method according to the invention may be implemented in automated incubator systems for culturing and monitoring embryos, such as human embryos. By implementing the present invention in such automated incubator systems, the selection processes and the quality control of e.g. culture media and other culturing conditions, may be more or less automated, i.e. fully manual with the software assisting the users with proposed decisions, semi-automatic or fully automatic with the incubator system making all the decisions, including early warnings, based on data analysis.

In a further aspect the invention relates to a system having means for carrying out the methods described above. Said system may be any suitable system, such as a computer comprising computer code portions constituting means for executing the methods as described above.

The system may further comprise means for acquiring images of the embryo at different time intervals, such as the system described in WO 2007/042044.

In a yet further aspect the invention relates to a data carrier comprising computer code portions constituting means for executing the methods as described above.

**Late stage monitoring**

The search for prognostic factors that predict embryo development and the outcome of in vitro fertilization (IVF) treatment have attracted considerable research attention as it is anticipated that knowledge of such factors may improve future IVF treatments. One promising predictive factor is the precise timing of key events in early embryo development. Studies that involve imaging have been limited to measurements of early development, such as pronuclear formation and fusion, and time to first cleavage (Nagy, Z.P. 1994, Fenwick, J. 2002, Lundin, K. 2001, Lemmen, J.G. 2008). An important finding of the time-lapse analysis is a correlation between the early cleavage pattern to the 4-cell stage and subsequent development to the blastocyst stage. Morphokinetic analysis on the development of bovine embryos have also been published, where timing, duration and intervals between cell cleavages in early embryo development successfully predicted subsequent development to the expanded blastocyst stage (Ramsing 2006, Ramsing 2007).

The present inventors have performed a large clinical study involving many human embryos and monitoring the development, not only until formation of a blastocyst, but further until sign of implantation of the embryo. In this study important differences in the temporal patterns of development between the embryos that implanted (i.e. embryos that were transferred and subsequently led to successful implantation) and those that did not (i.e. embryos that were transferred but did not lead to successful implantation) were observed. By using implantation as the endpoint, not only embryo competence for blastocyst formation, but also subsequent highly essential processes such as hatching and successful implantation in the uterus is assessed.

It has been found that there exists an optimal time range for parameters characterizing the embryonic cell divisions. The observations support the hypothesis that the viability of embryos is associated with a highly regulated sequence of cellular events that begin at the time of fertilization. In this clinical study on exclusively good quality embryos, it has been confirmed that an embryo's capability to implant is correlated with numerous different cellular events. The complexity, structure and parameters in the models must be adaptable to different clinical situations like incubation temperature, transfer times, culture media and other.

Timing of early events in embryonic development correlates with development into a blastocyst, and the development into a blastocyst is a necessity for a successful implantation and thus the formation of a blastocyst is a quality parameter in itself. However it has been found that the development into a blastocyst does not necessarily correlate with successful implantation of the embryo.

Supporters of early implantation at day 2 have argued that the extended culture of embryos to the blastocyst stage at around day 5 give rise to potential risks because the culture period is significantly prolonged which may disrupt embryo integrity. However, an extended culture period to the blastocyst stage has several advantages. Cultured human embryos have an average blastocyst formation rate of only approx. 30-50%, and by extending the culture period a large part of the low quality embryos have automatically been excluded, by not forming the blastocyst. Furthermore, after EGA at around the 5-8 blastomere stage the embryos own DNA controls the development. By evaluating the embryos at the blastocyst stage high quality embryos can be identified with a higher degree of certainty.

Thus, the data allows the detection of blastocyst related developmental criteria for implantation potential. The results in particular indicate that timing of late events, such as the onset of cavitation, are a consistently good indicator of implantation potential, and that the discrimination between implanting and non-implanting embryos is improved when using blastocyst quality criteria, e.g. tBI as opposed to the earlier events (t2, t3 and t4). Data indicate that incubating the embryos to the blastocyst stage can give additional important information that will improve the ability to select a viable embryo with high implantation potential. Furthermore, monitoring the embryos at the later stages up to the blastocyst stages may give better indication of quality issues and as shown in figs. 10-14 the implications of e.g. a toxic oil are more pronounced at the later stages. It may therefore be an advantage to include blastocyst quality criteria in the herein selected morphokinetic parameters, i.e. selected for quality surveillance.

The following list a number of embryo quality (selection) criteria and blastocyst quality criteria that may be applied singly or combined in groups to assess embryo quality and consequently asses quality issues when monitoring and automatically surveying culturing conditions.

*Multiple variables*

Multiple variables may be used when choosing selection criteria. When using multiple variables it can be an advantage that the variables are selected progressively such that initially one or more of the variables that can be determined early with a high accuracy are chosen, e.g. t2, t3, t4 or t5. Later other variables that can be more difficult to determine and is associated with a higher uncertainty can be used.

*Normalized or relative parameters*

It is unlikely that selection criteria derived from morphokinetic parameters would be universally applicable as the culturing conditions may vary from clinic to clinic. It may therefore be an advantage to define normalized morphokinetic parameters, e.g. normalized morphological parameters based on two, three, four, five or more parameters selected from the group of t2, t3, t4, t5, t6, t7 and t8. By normalizing the parameters the time of fertilization may be "removed" from the embryo quality assessment. Further, a normalized morphological embryo parameter may better describe the uniformity and/or regularity of the developmental rate of a specific embryo independent of the environmental conditions, because instead of comparing to "globally" determined absolute time intervals that may depend on the local environmental conditions, the use of normalized parameters ensure that specific ratios of time intervals can be compared to "globally" determined normalized parameters, thereby providing additional information of the embryo development. Further, the normalized morphokinetic parameters may provide additional regarding quality issues in the embryo development.

Thus, a blastocyst quality criterion may be to determine 1) the duration of a first time period from fertilization until translation of maternally inherited mRNA in the blastomeres is completed and 2) the duration of a second time period from initiation of transcription of the blastomeres own DNA to said blastocyst stage are determined, and wherein a blastocyst quality criterion is the ratio of said first and second time periods.

Thus, a blastocyst quality criterion may be to determine 1) the duration of a first time period from fertilization to a 5 blastomere embryo and 2) the duration of a second time period from the 5 blastomere embryo to said blastocyst stage are determined, and wherein a blastocyst quality criterion is the ratio of said first and second time periods.

The time from fertilization to the blastocyst stage is thereby divided into two time periods and the ratio between these time periods is a blastocyst quality criterion. The reason for dividing at the 5 blastomere stage is that this is approx. the time of embryonic gene activation. Thus, determine 1) the duration of a first time period from fertilization until translation of maternally inherited mRNA in the blastomeres is completed and 2) the duration of a second time period from initiation of transcription of the blastomeres own DNA to said blastocyst stage are determined, and wherein a blastocyst quality criterion is the ratio of said first and second time periods.

A corresponding blastocyst quality criterion can be provided by determining 1) the duration of a first time period from fertilization to blastocyst, and 2) the duration of a second time period from initiation of transcription of the blastomeres own DNA to said blastocyst stage and taking the ratio of these time periods. This ratio provides information on how much of the total time period from fertilization to blastocyst the embryo's own DNA is in control.

These ratios can be seen as measures of the relative development speed in a certain period relative to the overall development speed until that stage. Any variation in these ratios may indicate that the embryos are accelerating or decelerating in their development and this may be an indicator of quality issues in the culturing conditions.

The abovementioned blastocyst stage may be selected from the group of: initial compaction (IC), compaction/morula (M), initial differentiation of trophectoderm cells (IDT), early blastocyst (ERB), onset of cavitation/blastocyst (BI), expansion of blastocyst (EB), first contraction (CPS(1)), second contraction (CPS(2)), third contraction (CPS(3)), fourth contraction (CPS(4)), fifth contraction (CPS(5)), sixth contraction (CPS(6)), seventh contraction (CPS(7)), hatching (HB), and fully hatched (FH). Thus, tIC is the time from fertilization to initial compaction, tM is the time from fertilization to compaction/morula, etc.

*Examples of embryo quality criteria*

A blastocyst quality criterion is determination of $tB_i - t_i$ wherein $tB_i$ is selected from the group of {tM, tBI, tEB} and $t_i$ is selected from the group of {t5, t6, t7 and t8}.

A blastocyst quality criterion is determination of $(tB_i - t_i) / t_i$ wherein $tB_i$ is selected from the group of {tM, tBI, tEB} and $t_i$ is selected from the group of {t5, t6, t7 and t8}.

A blastocyst quality criterion is determination of $(tB_i - t_i) / tB_i$ wherein $tB_i$ is selected from the group of {tM, tBI, tEB} and $t_i$ is selected from the group of {t5, t6, t7 and t8}.

A blastocyst quality criterion is determination of the time from fertilization to a blastocyst stage.

A blastocyst quality criterion is determination of one or more of the following morphological blastocyst parameters:

tIC = time from fertilization to initial compaction,
tM = time from fertilization to compaction/morula,
tIDT = time from fertilization to initial differentiation of trophectoderm cells,
tERB = time from fertilization to early blastocyst,
tBI = time from fertilization to onset of cavitation,

tEB = time from fertilization to expansion of blastocyst,
tCPS(1) = time from fertilization to first contraction,
tCPS(2) = time from fertilization to second contraction,
tCPS(3) = time from fertilization to third contraction,
tHB = time from fertilization to hatching, and
tFH = time from fertilization to fully hatched.

A blastocyst quality criterion is determination of one or more of the following morphological blastocyst parameters:

tBI - tM,
tEB - tB,
tHE - tEB,
tEB - tM
tBI - tCPS(1),
tBI - tCPS(2),
tEB - tCPS(1),
tEB - tCPS(2),
tCPS(2) - tCPS(1),
tCPS(3) - tCPS(2).

A blastocyst quality criterion is determination of the absolute or relative 2D and/or 3D expansion of the blastocyst.
A blastocyst quality criterion is determination of the diameter and/or the volume of the embryo at the onset of expansion.
A blastocyst quality criterion is determination of the maximum diameter and/or the maximum volume of the blastocyst before hatching.
The normalized morphological embryo parameter may be selected from the group of

$$cc2/cc2\_3 = (t3-t2)/(t5-t2),$$

$$cc3/cc2\_3 = (t5-t3)/(t5-t2),$$

$$cc3/t5 = 1 - t3/t5,$$

$$s2/cc2 = (t4-t3)/(t3-t2),$$

$$s3/cc3 = (t8-t5)/(t5-t3),$$

and

$$cc2/cc3 = (t3-t2)/(t5-t3).$$

A quality criterion is determination of the extent of irregularity of the timing of cell divisions when the embryo develops from 4 to 8 blastomeres.
A quality criterion is determination of the maximum cleavage time for each blastomere when the embryo develops from 4 to 8 blastomeres.
A quality criterion is determination of the ratio between the maximum cleavage time for each blastomere when the embryo develops from 4 to 8 blastomeres and the duration of the total time period from 4 to 8 blastomeres; max(s3a,s3b,s3c)/s3.

*Combination with measurements of movement*

The quality criteria discussed above may also be combined with determinations of movement of the embryo, such as i) determining the extent and/or spatial distribution of cellular or organelle movement during the cell cleavage period; and/or ii) determining the extent and/or spatial distribution of cellular or organelle movement during the inter-cleavage period thereby obtaining an embryo quality measure.

Volumes within the zona pellucida that are devoid of movement (or similarly areas in a projected 2D image of the embryo that remain stationary) are an indication of "dead" zones within the embryo. The more and larger these immotile "dead" zones the lower the probability of successful embryo development. Large areas within a time-lapse series of embryo images without any type of movement (i.e. neither cellular nor organelle movement) indicates low viability. Organelle movement should generally be detectable in the entire embryo even when only comparing two or a few consecutive frames. Cellular movement may be more localized especially in the later phases of embryo development.

The cell positions are usually relatively stationary between cell cleavages (i.e. little cellular movement), except for a short time interval around each cell cleavage, where the cleavage of one cell into two leads to brief but considerable rearrangement of the dividing cells as well as the surrounding cells (i.e. pronounced cellular movement). The lesser movement between cleavages is preferred. Thus, any unexpected variation in cellular or organelle movement may be an indicator of quality issues in relation to the culturing conditions.

In relation to movement during cleavage and inter-cleavage periods we also refer to PCT application WO 2007/144001.

*Further examples of morphokinetic parameters*

As noted above, morphokinetic parameters to be used in accordance with certain embodiments of the invention may be established by combining a plurality of morphokinetic characteristics relating to the development of embryos. For example, in some implementations a morphokinetic parameter may be derived by obtaining values for a plurality of morphokinetic characteristics relating to the development of an embryo during an observation period, for example characteristics relating to any of cell cleavage times, time differences between pairs of cell divisions, and cell cycle durations. A value for a continuous variable may be determined by combining differences between the obtained values and corresponding reference values. The reference values may, for example, be determined from values for the plurality of characteristics obtained for one or more reference embryos of known development potential (e.g. KID positive embryos). A morphokinetic parameter may then be based on the determined value for the continuous variable.

Thus a method for determining a morphokinetic parameter for an embryo (referred to here as a study embryo) by generating a value for a continuous variable from a plurality of characteristics relating to the development of the embryo in accordance with an implementation of an embodiment of the invention may comprise various steps as follows: In a first step S1 a plurality of characteristics relating to the development of the study embryo during an observation period are obtained. These characteristics may fundamentally be based on cleavage times determined using conventional time-lapse embryonic imaging. One or more characteristics may be based on the timing of pronuclei fading / disappearance (tPNfading (or tPNf)).

In one example the characteristics may comprise a series of cell cycle durations $cc_i$ for a sequence of cell cycles. For example, the plurality of characteristics may comprise a series of values: $cc2a$ (= t3 - t2); $cc2b$ (= t4 - t2); $cc3a$ (= t5 - t3), $cc3b$ (= t6 -13), $cc3c$ (= t7 - t4), $cc3d$ (= t8 - t4). That is to say, for this example the sequence comprises durations for all cell cycles from $cc2a$ to $cc3d$ (i.e. all cell cycle durations up to an eight-blastomere embryo except for $cc1$). If there are particular cell cycle durations that are not measured for a given embryo (e.g. because the timing of a relevant cleavage event cannot be properly determined because of inadequate measurement or because the cleavage event has not occurred by the end of the incubation time (tEnd)), the missing cell cycle(s) may be left out of the sequence.

In a second step S2 average and variance values seen in a population of one or more reference embryos of known development potential (e.g. KID positive embryos) for characteristics corresponding to those obtained for the study embryo in step S1 are obtained. These may, for example, be read from a memory or other storage of an apparatus executing the method. The average and variance values may be obtained through retrospective analysis of images of embryos that proceeded to successful implantation. The embryos for which the average and variance values are obtained for a given study embryo may be referred to as reference embryos. The reference embryos may in some cases comprise embryos that have been expected at the same clinic as the study embryo, for example to help take account of inter-clinic variations associated with different incubation conditions. That is to say, step S2 may also comprise selecting an appropriate grouping of reference embryos for which to obtain the average and variance values based on characteristics of the study embryo. The average and variance values may be determined in accordance with conventional statistical analysis techniques, for example potentially involving the discarding of outlier data, and so forth. It will be appreciated the term "average" is used broadly herein to refer to a typical / representative / indicative value for a parameter seen in a sample population. In this regard, the average may, for example, correspond to a mean, mode or median value of the relevant characteristic for the reference population (positive KID population).

In a third step S3 a difference between the value of each characteristic seen for the study embryo and the corresponding average characteristic associated with the population of known development potential (e.g. KID positive) embryos is determined.

In a fourth step S4 a morphokinetic parameter for the study embryo (corresponding to a continuous variable) is determined by combining / aggregating the differences determined for each characteristic in a way which is weighted by the respective variance values. Thus in one specific example, a morphokinetic parameter (GIV) is defined as:

$$GIV = \frac{1}{n} \sqrt{\sum_{\substack{all\ i\ in \\ sequence}} \frac{(cci - cci_m)^2}{cci_v}}$$

Where cci is the series of cell cycle durations observed for the study embryo, $cci_m$ is the corresponding series of average cell cycle durations seen in a reference group of embryos (e.g. the positive KID population from patients under the age 35), and $cci_v$ are the corresponding variance values associated with the reference population. The parameter n is the number of cell cycle durations comprising the series cci. The differences (cci - $cci_m$) are normalized by the variance values $cci_v$ as part of the combining. This means that differences for particular cell cycles (values of i) which exhibit relatively high variance in the sample population contribute less to the value of GIV than differences for cell cycle which exhibit relatively low variance in the sample population. The differences (cci - $cci_m$) are squared in the combining, and this means the contribution to GIV is the same for a given difference, regardless of whether it is positive or negative (i.e. whether cci is longer or shorter than $cci_m$).

Generally speaking GIV is low when the study embryo exhibits a regular cleavage pattern and is high when the embryo exhibits an irregular cleavage pattern.

The particular morphokinetic parameter based on the particular sequence of cell cycle durations cci = cc2a; cc2b; cc3a; cc3b; cc3c and cc3d in this example may be referred to herein as a first generalized irregularity variable GIV1.

Thus, steps S1 to S4 discussed above represents a process for establishing a morphokinetic parameter for an embryo in accordance with an embodiment of the invention. It will be appreciated that similar methods may be used to establish a morphokinetic parameter for an embryo using different characteristics relating to the development of the study embryo and / or by combining the characteristics in a different way to generate a different morphokinetic parameter.

For example, while the first generalized irregularity variable GIV1 as described above is based on durations of cell cycles cc2a, cc2b, cc3a, cc3b, cc3c and cc3d (or at least the durations of the ones which are measured / not missing), other generalized irregularity variables may be based on durations of other cell cycles. For example, the following variations may be defined to provide different morphokinetic parameters for use in accordance with embodiments of the invention:

GIV2 (second generalized irregularity variable): similar to GIV1, but also including cc1, i.e. GIV2 may be calculated in a similar manner to GIV1, but based on cci = cc1, cc2a, cc2b, cc3a, cc3b, cc3c and cc3d.

GIV3 (third generalized irregularity variable): only including the second-generation cell cycles (cc2a and cc2b), i.e. GIV3 may be calculated in a similar manner to GIV1, but based on cci = cc2a and cc2b.

GIV4 (fourth generalized irregularity variable): only including the shortest cell cycle for the second- and third generations (cc2a and cc3a), i.e. GIV4 may be calculated in a similar manner to GIV1, but based on cci = cc2a and cc3a.

Whereas the above example generalized irregularity variables are based on cell cycle durations defined with respect to cell cleavage times, it will be appreciated that other generalized irregularity variables may be based on other timings (and / or durations between timings) which are associated with other embryonic developmental events. For example, a generalized irregularity variable used as a morphokinetic parameter in accordance with some embodiments of the invention may be established using timings defined relative to the time of pronuclei fading (tPNf). One example, which may be referred to as GIV5, can be defined as follows:

GIV5 (fifth generalized irregularity variable): comprising (t3 - tPNf) and cc3a.

In each case, if any of the characteristics comprising the generalized irregularity variable are missing for an embryo (e.g. because they were not properly measured or had not occurred before the end of incubation time), the corresponding characteristic may be left out of the calculation of the continuous variable (morphokinetic parameter), with the value of n being correspondingly reduced.

Other morphokinetic parameters may be determined from characteristics relating to the development of the study embryo other than cell cycle durations.

For example, a variation of the method discussed above with reference to steps S1 to S4 may comprise the following steps:

In a first step T1 the characteristics relating to the development of the study embryo may instead comprise a series of time differences Δtj between subsequent cell divisions (or morphological stages). For example, the plurality of characteristics may comprise a series of values: Δt1 (= t2); Δt2 (= t3 - t2); Δt3 (= t4 - t3); Δt4 (= t5 - t4), Δt5 (= t6 - t5), Δt6 (= t7 - t6), Δt7 (= t8 - t7) - i.e. the time differences between subsequent cell divisions up to the eight blastomeres stage. That is to say, for this example the sequence comprises all times between subsequent cell divisions from a single cell to an eight-blastomere embryo, or at least all of these times that are deemed to be properly measured (i.e. not missing).

In a second step T2 average and variance values seen in a population of one or more reference embryos of known development potential (e.g. KID positive embryos) for the characteristics obtained for the study embryo in step T1 are obtained.

In a third step T3 a difference between the value of each characteristic seen for the study embryo and the corresponding average characteristic associated with the population of known development potential (e.g. KID positive) embryos is determined. In a fourth step T4 a morphokinetic parameter for the study embryo (corresponding to a continuous variable) is determined by combining / aggregating the differences determined for each characteristic in a way which is weighted by the respective variance values. Thus in one specific example, a morphokinetic parameter (GTV) is defined as:

$$GTV = \frac{1}{k} \sum_{\substack{all\ j\ in \\ sequence}} \frac{\Delta tj - \Delta tj_m}{\Delta tj_v}$$

Where $\Delta tj$ is the series of differences in times for subsequent cell divisions observed for the study embryo, $\Delta tj_m$ is the corresponding series of average values seen in a reference group of embryos (e.g. the positive KID population from patients under the age 35), and $\Delta tj_v$ are the corresponding variance values associated with the reference population. The parameter k is the number of values comprising the series $\Delta tj$. The differences ($\Delta tj - \Delta tj_m$) are normalized by the variance values $\Delta tj_v$ as part of the combining. This means that differences for particular cell divisions (values of j) which exhibit relatively high variance in the sample population contribute less to the value of GTV than for those which exhibit relatively low variance in the sample population. In this example the contribution to GTV for each time difference depends on whether the difference in times for a particular pair of subsequent cell divisions is faster or slower than the average seen in the positive KID population (i.e. whether the difference is positive or negative).

This morphokinetic parameter GTV may be referred to herein as a generalized time variable, GTV. Generally speaking GTV is low when the study embryo exhibits a relatively fast development and is high when the embryo exhibits a relatively slow development.

The particular morphokinetic parameter based on the particular sequence Δt1 (= t2); Δt2 (= t3 - t2); Δt3 (= t4 - t3); Δt4 (= t5 - t4), Δt5 (= t6 - t5), Δt6 (= t7 - t6), Δt7 (= t8 - t7), as in this example, may be referred to herein as a third generalized time variable GIV3. It will again be appreciated that similar methods may be used to establish a morphokinetic parameter for an embryo using different characteristics relating to the development of the study embryo.

For example, while the third generalized time variable GTV3 as described above is based on all times between subsequent cell divisions from a single cell to an eight-blastomere embryo, other generalized irregularity variables may be based on other sequences of time differences. For example, the following variations may be defined to provide different morphokinetic parameters for use in accordance with embodiments of the invention:

GTV1 (first generalized time variable): similar to GTV3 but using only the time difference of the last two cell divisions observed up to the 8 blastomere state. I.e. Δti=(t8-t7), if t7 and t8 are annotated; or Δti = (t7-t6) if t8 is missing and t6 and t7 are annotated; or Δti=(t6-t5) if t8 and t7 are missing and t5 and t6 are annotated; or Δti=(t5-t4) if t8, t7 and t6 are missing and t4 and t5 are annotated; or Δti=(t4-t3) if t5 to t8 are missing and t3 and t4 are annotated; or Δti=(t3-t2) if t4 to t8 are missing and t2 and t3 are annotated; Δti= t2 if t3 to t8 are missing and t2 is annotated. In each case the parameter k is 1.

GTV2 (second generalized time variable): similar to GTV1 but with the later division time (cleavage time) in a pair replaced with tEnd (the end of the incubation time) where timings are missing. I.e. Δti=(t8-t7), if t7 and t8 are annotated; or Δti = (tEnd-t7) if t8 is missing and t7 is annotated; or Δti=(tEnd-t6) if t7 and t8 are missing and t6 is annotated; or Δti=(tEnd-t5) if t6 to t8 are missing and t5 is annotated; or Δti=(tEnd-t4) if t5 to t8 are missing and t4 is annotated; or Δti=(tEnd-t3) if t4 to t8 are missing and t3 is annotated; or Δti=(tEnd-t2) if t3 to t8 are missing and t2 is annotated. Mean and variance values for the reference population may be calculated based on Δti without substitution. For GTV2 the parameter k is always 1.

GTV3 (third generalized time variable): as discussed above, this morphokinetic parameter uses timings between all consecutive divisions that are not missing from the data to the 8 blastomere stage. i.e. Δti=((t8-t7), (t7-t6), (t6-t5), (t5-t4), (t4-t3), (t3-t2), t2). If a Δti from this sequence is missing for a particular embryo, it is omitted from the calculation. The parameter k is the number of Δti used in the calculation for a particular embryo.

GTV4 (fourth generalized time variable): similar to GTV3 but using all consecutive divisions with substitution of the last division time with incubation end time if missing. Δti=((t8-t7),(t7-t6),(t6-t5),(t5-t4),(t4-t3),(t3-t2),t2). If a ti is missing it is substituted with the tEnd. Mean and variance values for the reference population may be calculated based on Δti without substitution. K is the number of Δti used in the calculation for that particular embryo.

GTV5 (fifth generalized time variable): similar to GTV3 but using timings for the full cell cycles. I.e. Δti=((t8-t4), (t4-t2), t2). If a Δti is missing it is omitted, k is the number of Δti used in the calculation for a particular embryo.

GTV6 (sixth generalized time variable): similar to GTV5 but using all full cell cycles with substitution of the last division time with tEnd if missing. Δti=((t8-t4),(t4-t2), t2). If a ti is missing it is substituted with tEnd. Mean and variance is calculated based in the Δti without substitution. k is the number of Δti used in the calculation for that particular embryo.

GTV7 (seventh generalized time variable): similar to GTV2 but using only the period from insemination to the last annotated timing. I.e. Δti = t8 if t8 is annotated, Δti = t7 if t8 is missing and t7 is annotated, Δti = t6 if t7 and t8 are missing and t6 is annotated, and so on. For GTV7 the parameter k is always 1.

GTV8 (eighth generalized time variable): similar to GTV3 but using t8 if it is annotated and otherwise using tEnd if t8 is missing. For GTV8 the parameter k is always 1.

GTV9 (ninth generalized time variable): similar to GTV2 but using stages up to the blastocysts stage for evaluation on day 5 post insemination. Δti=(tB-tSB), if both tSB and tB are annotated; or Δti=(tEnd-tSB), if tB is missing and tSB is annotated; or Δti=(tEnd-tM), if tSB and tB is missing and tM is annotated; or Δti=(tM-t8), if tM to tB is missing and t8 is annotate; or Δti=(tEnd-t7), if t8 to tB is missing and t7 is annotated; or Δti=(tEnd-t6) if t7 to tB are missing and t6 is annotated; or Δti=(tEnd-t5) if t6 to tB are missing and t5 is annotated; or Δti=(tEnd-t4) if t5 to tB are missing and t4 is annotated; or Δti=(tEnd-t3) if t4 to tB are missing and t3 is annotated; or Δti=(tEnd-t2) if t3 to tB are missing and t2 is annotated. Mean and variance may be calculated based on the Δti without substitution. For GTV9 the parameter k is always 1.

GTV10 (tenth generalized time variable): similar to GTV4 but using all stages up to the blastocyst stage. For evaluation on day 5 post insemination. Δti=((tB-tSB),(tSB-tM),(tM-t8), (t8-t7),(t7-t6),(t6-t5),(t5-t4),(t4-t3),(t3-t2),t2). If a timing is missing it is substituted with the tEnd. Mean and variance is calculated based on the Δti without substitution. For GTV10 the parameter k is the number of Δti used in the calculation for that particular embryo.

## Embryo

In some cases the term "embryo" is used to describe a fertilized oocyte after implantation in the uterus until 8 weeks after fertilization at which stage it becomes a foetus. According to this definition the fertilized oocyte is often called a pre-embryo until implantation occurs. However, throughout this patent application we will use a broader definition of the term embryo, which includes the pre-embryo phase. It thus encompasses all developmental stages from the fertilization of the oocyte through morula, blastocyst stages hatching and implantation.

An embryo is approximately spherical and is composed of one or more cells (blastomeres) surrounded by a gelatine-like shell, the acellular matrix known as the zona pellucida. The zona pellucida performs a variety of functions until the embryo hatches, and is a good landmark for embryo evaluation. The zona pellucida is spherical and translucent, and should be clearly distinguishable from cellular debris.

An embryo is formed when an oocyte is fertilized by fusion or injection of a sperm cell (spermatozoa). The term is traditionally used also after hatching (i.e. rupture of zona pelucida) and the ensuing implantation. For humans the fertilized oocyte is traditionally called an embryo for the first 8 weeks. After that (i.e. after eight weeks and when all major organs have been formed) it is called a foetus. However the distinction between embryo and foetus is not generally well defined.

During embryonic development, blastomere numbers increase geometrically (1-2-4-8-16- etc.). Synchronous cell cleavage is generally maintained to the 16-cell stage in embryos. After that, cell cleavage becomes asynchronous and finally individual cells possess their own cell cycle. Human embryos produced during infertility treatment are usually transferred to the recipient before 16-blastomere stage. In some cases human embryos are also cultivated to the blastocyst stage before transfer. This is preferably done when many good quality embryos are available or prolonged incubation is necessary to await the result of a pre-implantation genetic diagnosis (PGD).

Accordingly, the term embryo is used in the following to denote each of the stages fertilized oocyte, zygote, 2-cell, 4-cell, 8-cell, 16-cell, morula, blastocyst, expanded blastocyst and hatched blastocyst, as well as all stages in between (e.g. 3 -cell or 5-cell)

## Examples

*Example 1*

Development for three different groups of mouse embryos incubated in three different temperatures of the incubation medium were investigated under similar conditions, i.e. only the temperature differed between the three different groups. The temperature of the incubation media was assessed by measuring the temperature of the slideholder using a YSI precision thermometer.

The three different temperatures were 36.5°C (33 embryos), 37.5°C (63 embryos) and 38.5°C (35 embryos), respectively. Nearly all mouse embryos reached the blastocyst stage as seen in the below table.

| Temperature of slide holder (°C) | N | Blastocyst rate (%) |
|---|---|---|
| 36.5 | 33 | 100 |
| 37.5 | 63 | 98 |
| 38.5 | 35 | 100 |

The table below shows the measured average timing for different cell divisions, the morula and blastocyst stage.

| Temp. (°C) | 2 cells (t2) | 3 cells (t3) | 4 cells (t4) | 5 cells (t5) | 6 cells (t6) | 7 cells (t7) | 8 cells (t8) | 9+ cells (t9) | Morula | Blastocyst |
|---|---|---|---|---|---|---|---|---|---|---|
| 36.5 | 4.61 | 26.36 | 27.57 | 35.91 | 36.30 | 37.10 | 37.54 | 44.54 | 49.45 | 67.46 |
| 37.5 | 3.75 | 23.43 | 24.25 | 32.12 | 32.54 | 33.19 | 33.63 | 40.72 | 45.85 | 59.27 |
| 38.5 | 3.06 | 21.96 | 22.50 | 30.62 | 30.97 | 31.43 | 31.87 | 39.06 | 44.29 | 55.03 |

These data have been plotted in three graphs shown in fig. 5. The difference between various cell divisions is shown in fig. 6. The data and the graphs show that increasing the temperature of the medium clearly speeds up the development.

In order to assess the difference in development a relative rate coefficient $k$ can be defined. If $k$ is set to 1 at base temperature ($T_b$) the following relationship can be assumed:

$$k(T) = 1+\alpha^*(T_b -36.5)$$

where $T$ is the temperature in °C and $\alpha$ is the temperature dependency coefficient.

The expected time $t$ for a given temperature $T$, relative to $t(T_b)$, is inversely proportional to $k(T)$:

$$t(T) = t(T_b)/k(T)$$

The above linear simplification offers the advantage of only requiring the estimation of a single parameter. Conversely, it is probably only valid within a narrow temperature range. However, in the case of human embryo incubation, the expected maximum temperature span would be somewhat below ± 1°C, such that the practical influence of non-linearity can be considered negligible.

Optimising $k(T)$ and $t(T)$ by utilisation of the above listed mouse embryo data, using the time of division to 5 cells (t5), $\alpha$ is estimated to 0.080 ±0.015 (95 % CI).

The $Q_{10}$ value is calculated as:

$$Q_{10} = \left(\frac{R_2}{R_1}\right)^{10/(T_2-T_1)}$$

where $R$ is the rate and $T$ is the temperature.

Utilising the above parameter, the mouse embryo data, and the ± 1 °C span in the experiment, the above equation yields a $Q_{10}$ of 2.22, which is inside the normally expected range of 2 - 3 in biological systems (Reyes et al., 2008, Mammalian peripheral circadian oscillators are temperature compensated. J. Biol. Rhythms 23: 95-98).

The same calculations have been performed for a set of data from 1397 human embryos extracted from different clinics. The incubation conditions for these human embryos are therefore not as similar as the above mentioned mouse embryos. However, the clinics belong to the same chain of IVF clinics using the same instrumentation. All embryos have been transferred with homogenised procedures, besides temperature. Utilising t5 here again, and optimising according to $k(T)$ and $t(T)$, the estimate for $\alpha$ becomes 0.058 $\pm$ 0.028 (95 % CI).

In contrast to the mouse embryos these human embryos have been incubated under slightly different conditions. The extracted human embryo data are therefore not comparable to the same degree as the mouse embryo data. However, again the data from the human embryos indicate that a higher temperature of the medium speeds up the development. By constantly monitoring the morphokinetic parameters this increase in development speed may be detected and provide an early warning of a problem. This problem may be related to an undesirable increase in temperature, which may be corrected before any embryos are actually transferred.

**Claims**

1. A computer implemented method for automatically detecting variations and/or abnormalities in the culturing conditions of in vitro incubating embryos, the method comprising the steps of:

   a) obtaining a first dataset comprising morphokinetic parameters relating to the development of a first group of embryos,
   b) obtaining a second dataset comprising corresponding morphokinetic parameters relating to the development of a second group of embryos,
   c) modifying the first and second datasets by extracting morphokinetic parameter outliers from the first dataset and or the second datasets,
   d) calculating the difference between specific morphokinetic parameters from the modified first dataset and the corresponding morphokinetic parameters from the modified second dataset, and
   e) monitoring said morphokinetic difference thereby detecting variations in the culturing conditions of the first and second group of embryos, and issuing a warning when the morphokinetic difference is above a predefined level.

2. The method according to any of the preceding claims, wherein steps a) - d) are repeated and the second dataset is continuously updated with the most recent embryo data.

3. The method according to claim 1, wherein said predefined level is determined as a predefined level above the standard deviation of one or more of the morphokinetic parameters from the first dataset.

4. The method according to any of the preceding claims, wherein the morphokinetic parameter outliers are excluded from the datasets.

5. The method according to any of the preceding claims, wherein all morphokinetic parameters from embryos with one or more morphokinetic parameter outliers are excluded from the datasets.

6. The method according to any of the preceding claims, wherein the morphokinetic parameters are selected from the group of:

   - the time of cleavage to a n blastomere embryo, tn, where n = {1, ..., 8},
   - duration of cell cycles cc1, cc2, cc2b, cc3, cc2_3 and cc4,
   - synchronicities s2, s3, s3a, s3b and s3c,
   - number of blastomeres at specific predefined timepoints,
   - timing, extent or duration of cellular and/or organelle movement,
   - any combination of these morphokinetic parameters.

7. The method according to any of the preceding claims, further comprising the step of calculating running mean values or running median values of the morphokinetic parameters.

8. The method according to any of the preceding claims, further comprising the step of calculating mean (average) values, median values, variance values and/or standard deviation values of the morphokinetic.

9. The method according to any of the preceding claims, wherein the following is a morphokinetic parameter:

$$CC_{norm} = \frac{1}{n}\sqrt{\sum_{all\ i}\left(\frac{CCi - CCi_m}{CCi_v}\right)^2}$$

wherein $i = \{1,2,3,4\}$, $cci_m$ is the mean value or median value of $cci$ and $cci_v$ is the variance of cci.

10. The method according to any of the preceding claims, wherein at least one morphokinetic parameter for an embryo is established by obtaining values for a plurality of characteristics relating to the development of the embryo during an observation period; and determining a value for the at least one morphokinetic parameter by combining differences between the obtained values and corresponding reference values for the plurality of characteristics in a pre-defined manner.

11. The method according to any of the preceding claims, wherein at least one of the morphokinetic parameters is based on a generalised irregularity variable, GIV, determined according to:

$$GIV = \frac{1}{n}\sqrt{\sum_{\substack{all\ i\ in \\ sequence}}\frac{(cci - cci_m)^2}{cci_v}}$$

where cci is a series of cell cycle durations for an embryo, $cci_m$ is a corresponding series of average cell cycle durations seen in a reference population of embryos, $cci_v$ is a corresponding series of variance values for the respective cell cycle durations seen in the reference population, and n is the number of cell cycle durations comprising the series of cell cycle durations,
or
wherein at least one of the morphokinetic parameters is based on a generalised time variable, GTV, determined according to:

$$GTV = \frac{1}{k}\sum_{\substack{all\ j\ in \\ sequence}}\frac{\Delta tj - \Delta tj_m}{\Delta tj_v}$$

where $\Delta tj$ is a series of differences in times for subsequent cell divisions for an embryo, $\Delta tj_m$ is a corresponding series of average values seen in a reference population of embryos, $\Delta tj_v$ is a corresponding series of variance values for the respective differences in times for subsequent cell divisions seen in the reference population, and k is the number of differences in times comprising the series.

12. The method according to any of the preceding claims, wherein morphokinetic outliers are selected from the group of: directly cleaving 1→3 cell embryos with cc2<5 hours, directly cleaving 2→5 cell embryos, short cell cycle embryos, long cell cycle embryos, and embryos experiencing multinuclarity.

13. A computer comprising computer code portions constituting means for executing the methods according to claim 1 to 11.

**Patentansprüche**

1. Computerimplementiertes Verfahren zum automatischen Detektieren von Variationen und/oder Abnormalitäten in den Kulturbedingungen der in vitro-Inkubierung von Embryonen, wobei das Verfahren die Schritte umfasst:

   a) Erhalten eines ersten Datensatzes, der morphokinetische Parameter umfasst, die die Entwicklung einer ersten Gruppe von Embryonen betreffen,
   b) Erhalten eines zweiten Datensatzes, der entsprechende morphokinetische Parameter umfasst, die die Entwicklung einer zweiten Gruppe von Embryonen betreffen,
   c) Modifizieren des ersten und des zweiten Datensatzes durch Extrahieren von Ausreißern der morphokinetischen Parameter aus dem ersten Datensatz und/oder den zweiten Datensätzen,
d) Berechnen der Differenz zwischen spezifischen morphokinetischen Parametern aus dem modifizierten ersten Datensatz und den entsprechenden morphokinetischen Parametern aus dem modifizierten zweiten Datensatz, und
e) Überwachen der morphokinetischen Differenz, wodurch Variationen in den Kultivierungsbedingungen der ersten und zweiten Gruppe von Embryonen detektiert werden, und Ausgeben einer Warnung, wenn die morphokinetische Differenz oberhalb eines vordefinierten Niveaus liegt.

2. Verfahren nach einem der vorhergehenden Ansprüche, wobei Schritte a) bis d) wiederholt werden und der zweite Datensatz kontinuierlich mit den neuesten Embryodaten aktualisiert wird.

3. Verfahren nach Anspruch 1, wobei das vordefinierte Niveau als vordefiniertes Niveau über der Standardabweichung von einem oder mehreren der morphokinetischen Parameter aus dem ersten Datensatz definiert wird.

4. Verfahren nach einem der vorhergehenden Ansprüche, wobei die Ausreißer der morphokinetischen Parameter aus den Datensätzen ausgeschlossen werden.

5. Verfahren nach einem der vorhergehenden Ansprüche, wobei alle morphokinetischen Parameter von Embryonen mit einem oder mehreren Ausreißern der morphokinetischen Parameter aus den Datensätzen ausgeschlossen werden.

6. Verfahren nach einem der vorhergehenden Ansprüche, wobei die morphokinetischen Parameter ausgewählt sind aus der Gruppe von:

   - der Zeit der Teilung zu einem n Blastomer-Embryo, tn, wobei $n = \{1, ..., 8\}$,
   - der Dauer der Zellzyklen cc1, cc2, cc2b, cc3, cc2_3 und cc4,
   - Synchronizitäten s2, s3, s3a, s3b und s3c,
   - Anzahl der Blastomeren an spezifischen vordefinierten Zeitpunkten,
   - Timing, Ausmaß oder Dauer von zellulärer und/oder Organellenbewegung,
   - jeglicher Kombination dieser morphokinetischen Parameter.

7. Verfahren nach einem der vorhergehenden Ansprüche, des Weiteren umfassend den Schritt des Berechnens gleitender Mittelwerte oder gleitender Medianwerte der morphokinetischen Parameter.

8. Verfahren nach einem der vorhergehenden Ansprüche, des Weiteren umfassend den Schritt des Berechnens von Mittelwerten (Durchschnittswerten), Medianwerten, Varianzwerten und/oder Standardabweichungswerten der morphokinetischen Parameter.

9. Verfahren nach einem der vorhergehenden Ansprüche, wobei das folgende ein morphokinetischer Parameter ist:

$$CC_{norm} = \frac{1}{n} \sqrt{\sum_{alle\ i} \left( \frac{CCi - CCi_m}{CCi_v} \right)^2}$$

wobei $i = \{1, 2, 3, 4\}$, $cci_m$ der Mittelwert oder Medianwert von cci ist, und $cci_v$ die Varianz von cci ist.

10. Verfahren nach einem der vorhergehenden Ansprüche, wobei mindestens ein morphokinetischer Parameter für einen Embryo hergeleitet wird, indem Werte für eine Vielzahl von Charakteristika erhalten werden, welche die Entwicklung des Embryos während einer Beobachtungsperiode betreffen; und ein Wert für den mindestens einen morphokinetischen Paramter ermittelt wird, indem Differenzen zwischen den erhaltenen Werten und entsprechenden Referenzwerten für die Vielzahl der Charakteristika in einer vordefinierten Weise kombiniert werden.

11. Verfahren nach einem der vorhergehenden Ansprüche, wobei mindestens einer der morphokinetischen Parameter auf einer generalisierten Ungleichförmigkeitsvariablen, GIV, basiert, die ermittelt wird gemäß:

$$GIV = \frac{1}{n}\sqrt{\sum_{\substack{alle\ i\ in \\ Folge}} \frac{(cci - cci_m)^2}{cci_v}}$$

wobei cci eine Reihe von Zellzyklusdauern für einen Embryo ist, $cci_m$ eine entsprechende Reihe von durchschnittlichen Zellzyklusdauern ist, die man in einer Referenzpopulation von Embryonen sieht, $cci_v$ eine entsprechende Reihe von Varianzwerten für die jeweiligen Zellzyklusdauern ist, die man in der Referenzpopulation sieht, und n die Zahl der Zellzyklusdauern ist, die die Reihe von Zellzyklusdauern umfassen,
oder

$$GTV = \frac{1}{k}\sum_{\substack{alle\ j\ in \\ Folge}} \frac{\Delta tj - \Delta tj_m}{\Delta tj_v}$$

wobei mindestens einer der morphokinetischen Parameter auf einer generalisierten Zeitvariablen, GTV, basiert, die ermittelt wird gemäß:
wobei $\Delta tj$ eine Reihe von zeitlichen Differenzen für nachfolgende Zellteilungen für einen Embryo ist, $\Delta tj_m$ eine entsprechende Reihe von Durchschnittswerten ist, die man in einer Referenzpopulation von Embryonen sieht, $\Delta tj_v$ eine entsprechende Reihe von Varianzwerten für die jeweiligen zeitlichen Differenzen für nachfolgende Zellteilungen ist, die man in der Referenzpopulation sieht, und k die Anzahl von zeitlichen Differenzen ist, welche die Reihe umfassen.

12. Verfahren nach einem der vorhergehenden Ansprüche, wobei morphokinetische Ausreißer ausgewählt sind aus der Gruppe: direkte Teilung von 1→3 Zell-Embryonen mit cc2 < 5 Stunden, direkte Teilung von 2→5 Zell-Embryonen, Embryonen mit kurzem Zellzyklus, Embryonen mit langem Zellzyklus, und Embryonen, die Multinuklearität aufweisen.

13. Computer, der Computercodeabschnitte umfasst, die Mittel zum Ausführen der Verfahren gemäß den Ansprüchen 1 bis 11 bilden.

**Revendications**

1. Procédé implémenté par ordinateur permettant de détecter automatiquement des variations et/ou des anomalies dans les conditions de mise en culture d'embryons en train d'incuber in vitro, le procédé comportant les étapes consistant à :

   a) obtenir un premier ensemble de données comprenant des paramètres morphocinétiques relatifs au développement d'un premier groupe d'embryons,
   b) obtenir un second ensemble de données comprenant des paramètres morphocinétiques correspondants relatifs au développement d'un second groupe d'embryons,
   c) modifier les premier et second ensembles de données en extrayant des valeurs aberrantes de paramètres morphocinétiques du premier ensemble de données et/ou du second ensemble de données,
   d) calculer la différence entre des paramètres morphocinétiques spécifiques provenant du premier ensemble de données modifié et les paramètres morphocinétiques correspondants provenant du second ensemble de données modifié, et
   e) surveiller ladite différence morphocinétique, ce qui permet de détecter des variations dans les conditions de mise en culture du premier et du second groupe d'embryons, et émettre un avertissement lorsque la différence morphocinétique est supérieure à un niveau prédéfini.

2. Procédé selon l'une quelconque des revendications précédentes, dans lequel les étapes a) à d) sont répétées et le second ensemble de données est mis à jour en continu avec les données embryonnaires les plus récentes.

3. Procédé selon la revendication 1, dans lequel ledit niveau prédéfini est déterminé en tant que niveau prédéfini au-dessus de l'écart-type d'un ou de plusieurs des paramètres morphocinétiques provenant du premier ensemble de données.

4. Procédé selon l'une quelconque des revendications précédentes, dans lequel les valeurs aberrantes de paramètres morphocinétiques sont exclues des ensembles de données.

5. Procédé selon l'une quelconque des revendications précédentes, dans lequel tous les paramètres morphocinétiques provenant d'embryons ayant une ou plusieurs valeurs aberrantes de paramètres morphocinétiques sont exclus des ensembles de données.

6. Procédé selon l'une quelconque des revendications précédentes, dans lequel les paramètres morphocinétiques sont choisis dans le groupe consistant en :

   - le temps de clivage en un embryon à n blastomères, tn, où n = {1, ..., 8},
   - une durée de cycles cellulaires cc1, cc2, cc2b, cc3, cc2_3 et cc4,
   - des synchronicités s2, s3, s3a, s3b et s3c,
   - le nombre de blastomères à des instants prédéfinis spécifiques,
   - une temporisation, une étendue ou une durée d'un mouvement cellulaire et/ou d'un mouvement d'organelle,
   - une quelconque combinaison de ces paramètres morphocinétiques.

7. Procédé selon l'une quelconque des revendications précédentes, comportant en outre l'étape consistant à calculer des valeurs moyennes mobiles ou des valeurs médianes mobiles des paramètres morphocinétiques.

8. Procédé selon l'une quelconque des revendications précédentes, comportant en outre l'étape consistant à calculer des valeurs moyennes, des valeurs médianes, des valeurs de variance et/ou des valeurs d'écart-type des paramètres morphocinétiques.

9. Procédé selon l'une quelconque des revendications précédentes, dans lequel ce qui suit est un paramètre morphocinétique :

$$CC_{norm} = \frac{1}{n} \sqrt{\sum_{tous\ les\ i} \left( \frac{CCi - CCi_m}{CCi_v} \right)^2}$$

où $i = \{1,2,3,4\}$, $cci_m$ est la valeur moyenne ou la valeur médiane de $cci$ et $cci_v$ est la variance de $cci$.

10. Procédé selon l'une quelconque des revendications précédentes, dans lequel au moins un paramètre morphocinétique pour un embryon est établi en obtenant des valeurs pour une pluralité de caractéristiques relatives au développement de l'embryon pendant une période d'observation ; et en déterminant une valeur pour l'au moins un paramètre morphocinétique en combinant des différences entre les valeurs obtenues et des valeurs de référence correspondantes pour la pluralité de caractéristiques d'une manière prédéfinie.

11. Procédé selon l'une quelconque des revendications précédentes, dans lequel au moins un des paramètres morphocinétiques est basé sur une variable d'irrégularité généralisée, GIV, déterminée en fonction de :

$$GIV = \frac{1}{n} \sqrt{\sum_{tous\ les\ i\ en\ séquence} \frac{(cci - cci_m)^2}{cci_v}}$$

où $cci$ est une série de durées de cycles cellulaires pour un embryon, $cci_m$ est une série correspondante de durées de cycles cellulaires moyennes constatées dans une population d'embryons de référence, $cci_v$ est une série correspondante de valeurs de variance pour les durées de cycles cellulaires respectives constatées dans la population de référence, et n est le nombre de durées de cycles cellulaires comprenant les séries de durées de cycles cellulaires, ou

dans lequel au moins un des paramètres morphocinétiques est basé sur une variable temporelle généralisée, GTV, déterminée en fonction de :

$$GTV = \frac{1}{k} \sum_{\substack{tous\ les\ j \\ en\ séquence}} \frac{\Delta tj - \Delta tj_m}{\Delta tj_v}$$

où $\Delta tj$ est une série de différences temporelles pour des divisions cellulaires ultérieures pour un embryon, $\Delta tj_m$ est une série correspondante de valeurs moyennes constatées dans une population d'embryons de référence, $\Delta tj_v$ est une série correspondante de valeurs de variance pour les différences temporelles respectives pour des divisions cellulaires ultérieures constatées dans la population de référence, et k est le nombre de différences temporelles comprenant la série.

12. Procédé selon l'une quelconque des revendications précédentes, dans lequel des valeurs aberrantes morphocinétiques sont choisies dans le groupe consistant en : embryons à clivage direct 1→3 cellules avec cc2 < 5 heures, embryons à clivage direct 2→5 cellules, embryons à cycle cellulaire court, embryons à cycle cellulaire long, et embryons connaissant une multinucléarité.

13. Ordinateur comprenant des parties de code informatique constituant des moyens pour exécuter les procédés selon les revendications 1 à 11.

Fig. 1

Kinetics

a) Compaction/Morula
b) Initial differentiation of trophectoderm (IDT)
c) Onset of cavitation/early blastocyst/blastocyst (B)
d) Onset of expansion (OE)
e) Hatching Blastocyst (HB)

Morphology
t5-a) Interval where Initial Compaction (IC) can be observed if
present usually IC preceeds "a" by minutes to a few hours.
a-c) Interval where "Partial compaction" (PC) can be observed if
present.
a-d+) interval where "Degree of vacuolization" (VC(X)) and
number of contractions (NC(X)) can be observed if present.

Fig. 2

EP 2 890 781 B1

Fig. 3

EP 2 890 781 B1

Fig. 4a

Fig. 4b

Fig. 5

Fig. 6

**Annotated**

LCC 14%

SCC 26%

MCC 60%

Fig. 7a

**Transferred**

LCC 9%

SCC 14%

MCC 77%

Fig. 7b

8,3%  17,7%  7,2%  15,4%

SCC  MCC  LCC  All

Fig. 7c

Fig. 8

Fig. 9

Frozen one-cell embryos from hybrid mice

One cell embryos (n=10/trt)

6 groups of TX-100 (0 to 0.002%) in 25 ul media

HTF without protein

6 groups of cumene peroxide (0 to 10 µM) in oil in 10 ul media

Expanded blastocysts vs morphokinetic analysis

Fig. 10

Fig. 11

Fig. 12

Fig. 13

Fig. 14

**EP 2 890 781 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2282210 A
- EP 2013063240 W
- DK 2012050236 W
- DK 201205018 W
- EP 12174432
- WO 2007042044 A
- WO 2007144001 A

### Non-patent literature cited in the description

- Linking successful implantation with the exact timing of cell division events obtained by time-lapse system in the embryoscope. **HERRERO J et al.** Fertility And Sterility. Elsevier Science Inc, vol. 94, 149
- Kinetic markers of human embryo quality using time-lapse recordings of IVF/ICSI-fertilized oocytes. **LEMMEN J G et al.** Reproductive Biomedicine Online. Reproductive Healthcare Ltd, vol. 17, 385-391
- **M. MESEGUER et al.** The Use of Morphokinetics as a Predictor of Embryo Implantation. *Human Reproduction,* ISSN 0268-1161, 2658-2671
- **MARIABEATRICE DAL CANTO et al.** Cleavage kinetics analysis of human embryos predicts development to blastocyst and implantation. *Reproductive Biomedicine Online,* 02 August 2012, vol. 25 (5), ISSN 1472-6483, 474-480
- **CONNIE WONG et al.** Non-invasive imaging of human embryos before embryonic genome activation predicts development to the blastocyst stage. *Nature Biotechnology,* 01 October 2010, vol. 28 (10), ISSN 1087-0156, 1115-1121
- *Determination of a change in a cell population,* 16 October 2006
- **REYES et al.** Mammalian peripheral circadian oscillators are temperature compensated. *J. Biol. Rhythms,* 2008, vol. 23, 95-98